# EUROPEAN PATENT SPECIFICATION

(11) **EP 0 877 624 B1**
(45) Date of publication and mention of the grant of the patent: **31.03.2004**
(21) Application number: 97902076.5
(22) Date of filing: 21.01.1997
(51) Int. Cl.: A61K 39/09

(54) **STREPTOCOCCAL C5A PEPTIDASE VACCINE**
STREPTOKOKKEN C5A PEPTIDASE IMPFSTOFF
VACCIN A BASE DE PEPTIDASE C5a DU STREPTOCOQUE

(30) Priority: 22.01.1996 US 589756
(43) Date of publication of application: 18.11.1998
(73) Proprietor: REGENTS OF THE UNIVERSITY OF MINNESOTA, Minneapolis MN 55455 (US)
(72) Inventor: CLEARY, Paul, P., Shoreview, MN 55112 (US)
(74) Representative: Grant, Anne Rosemary
(86) International application number: PCT/US1997/001056
(87) International publication number: WO 1997/026008

(56) References cited:
- WO-A-93/14198
- WO-A-93/21220
- WO-A-95/28960
- THE JOURNAL OF BIOLOGICAL CHEMISTRY, vol. 265, 1990, pages 3161--3167, XP000653803 CHEN C.C. AND CLEARY P.P.: "COMPLETE NUCLEOTIDE SEQUENCE OF THE STREPTOCOCCAL C5a PEPTIDASE GENE OF STREPTOCOCCUS PYOGENES" cited in the application
- THE JOURNAL OF INFECTIOUS DISEASES, vol. 163, 1991, pages 109-116, XP000653800 O'CONNOR S.P. ET AL: "THE HUMAN ANTIBODY RESPONSE TO STREPTOCOCCAL C5a PEPTIDASE" cited in the application
- INFECTION AND IMMUNITY, vol. 60, 1992, pages 4239-4244, XP000653845 CLEARY P.P. ET AL.: "SIMILARITY BETWEEN THE GROUP B AND A STREPTOCOCCAL C5a PEPTIDASE GENES" cited in the application

## Description

### Background of the Invention

There are several different β-hemolytic streptococcal species that have been identified. *Streptococcus pyogenes*, also called group A streptococci, is a common bacterial pathogen of humans. Primarily a disease of children, it causes a variety of infections including pharyngitis, impetigo and sepsis in humans. Subsequent to infection, autoimmune complications such as rheumatic fever and acute glomerulonephritis can occur in humans. This pathogen also causes severe acute diseases such as scarlet fever, necrotizing fasciitis and toxic shock.

Sore throat caused by group A streptococci, commonly called "strep throat," accounts for at least 16% of all office calls in a general medical practice, depending on the season. Hope-Simpson, E., "Streptococcus pyogenes in the throat: A study in a small population, 1962-1975," J. Hyg. Camb., 87:109-129 (1981). This species is also the cause of the recent resurgence in North America and four other continents of toxic shock associated with necrotizing fasciitis. Stevens, D. L., "Invasive group A streptococcus infections," Clin. Infect. Dis., 14:2-13 (1992). Also implicated in causing strep throat and occasionally in causing toxic shock are groups C and G streptococci. Hope-Simpson, E., "Streptococcus pyogenes in the throat: A study in a small population, 1962-1975," J. Hyg. Camb., 87:109-129(1981).

Group B streptococci, also known as *Streptococcus agalactiae*, are responsible for neonatal sepsis and meningitis. T.R. Martin et al., "The effect of type-specific polysaccharide capsule on the clearance of group B streptococci from the lung of infant and adult rats", J. Infect Dis., 165:306-314 (1992). Although frequently a member of vaginal mucosal flora of adult females, from 0.1 to 0.5/1000 newborns develop serious disease following infection during delivery. In spite of the high mortality from group B streptococcal infections, mechanisms of the pathogenicity are poorly understood. Martin, T. R., et al., "The effect of type-specific polysaccharide capsule on the clearance of Group B streptococci from the lung of infant and adult rats," J. Infect. Dis., 165:306-314 (1992).

Streptococcal infections are currently treated by antibiotic therapy. However, 25-30% of those treated have recurrent disease and/or shed the organism in mucosal secretions. At present no means is available to prevent streptococcal infections. Historically, streptococcal vaccine development has focused on the bacterium's cell surface M protein. Bessen, D., et al., "Influence of intranasal immunization with synthetic peptides corresponding to conserved epitopes of M protein on mucosal colonization by group A streptococci," Infect. Immun., 56:2666-2672 (1988); Bronze, M. S., et al., "Protective immunity evoked by locally administered group A streptococcal vaccines in mice," Journal of Immunology, 141:2767-2770 (1988).

Two major problems will limit the use, marketing, and possibly FDA approval, of a M protein vaccine. First, more than 80 different M serotypes of *S. pyogenes* exist and new serotypes continually arise. Fischetti, V. A., "Streptococcal M protein: molecular design and biological behavior, Clin. Microbiol. Rev., 2:285-314 (1989). Thus, inoculation with one serotype-specific M protein will not likely be effective in protecting against other M serotypes. The second problem relates to the safety of an M protein vaccine. Several regions of the M protein contain antigenic epitopes which are immunologically cross-reactive with human tissue, particularly heart tissue. The N-termini of M proteins are highly variable in sequence and antigenic specificity. Inclusion of more than 80 different peptides, representing this variable sequence, in a vaccine would be required to achieve broad protection against group A streptococcal infection. New variant M proteins would still continue to arise, requiring ongoing surveillance of streptococcal disease and changes in the vaccine composition. In contrast, the carboxyl-termini of M proteins are conserved in sequence. This region of the M protein, however, contains an amino acid sequence which is immunologically cross-reactive with human heart tissue. This property of M protein is thought to account for heart valve damage associated with rheumatic fever. P. Fenderson et al., "Tropomyosinsharies immunologic epitopes with group A streptococcal M proteins, J. Immunol. 142:2475-2481 (1989). In an early trial, children who were vaccinated with M protein in 1979 had a ten fold higher incidence of rheumatic fever and associated heart valve damage. Massell, B. F., et al., "Rheumatic fever following streptococcal vaccination, JAMA, 207:1115-1119 (1969).

Other proteins under consideration for vaccine development are the erythrogenic toxins, streptococcal pyrogenic exotoxin A and streptococcal pyrogenic exotoxin B. Lee, P. K., et al., "Quantification and toxicity of group A streptococcal pyrogenic exotoxins in an animal model of toxic shock syndrome-like illness," J. Clin. Microb., 27:1890-1892 (1989). Immunity to these proteins could prevent the deadly symptoms of toxic shock, but will not prevent colonization by streptococci, nor likely lower the incidence of strep throat. Estimates suggest that the incidence of toxic shock infections is 10 to 20 cases per 100,000 population; therefore, use of these proteins to immunize the general population against toxic shock is neither practical nor economically feasible.

Thus, there remains a continuing need for an effective means to prevent or ameliorate streptococcal infections. More specifically, a need exists to develop compositions useful in vaccines to prevent or ameliorate colonization of host tissues by streptococci, thereby reducing the incidence of strep throat and impetigo. Elimination of sequelae such as rheumatic fever, acute glomerulonephritis, sepsis, toxic shock and necrotizing fasciitis would be a direct consequence of reducing the incidence of acute infection and carriage of the organism. A need also exists to develop compositions useful in vaccines to prevent or ameliorate infections caused by all β-hemolytic streptococcal species, namely groups A, B, C and G.

### Summary of the Invention

The present invention provides a vaccine, and methods of vaccination, effective to prevent or reduce the incidence of β-hemolytic *Streptococcus* in susceptible mammals, including humans, and domestic animals such as dogs, cows, pigs and horses. The vaccine comprises an immunogenic amount of an enzymatically inactive streptococcal C5a peptidase (SCP), or one or more immunogenic fragments or mutants thereof in combination with a physiologically-acceptable, non-toxic vehicle. It may also contain an immunological adjuvant. The vaccine can be used to prevent colonization of group A *Streptococcus,* group B *Streptococcus*, group C *Streptococcus* or group G *Streptococcus.* The vaccine may comprise an immunogenic recombinant enzymatically inactive streptococcal C5a peptidase conjugated or linked to an immunogenic peptide or to an immunogenic polysaccharide.

The streptococcal C5a peptidase vaccine can be administered by subcutaneous or intramuscular injection. Alternatively, the vaccine can be administered by oral ingestion or intranasal inoculation.

As described in the working examples below, an SCP gene (*scpA49*) was cloned into an *E*. *coli* expression vector (pGEX-4T-1). The transferase-SCP fusion from the *E. coli* clone was expressed and purified. The purified recombinant SCP was then used to immunize mice. The vaccinated mice and a control group of mice were then challenged with wild-type *Streptococci*. The mice receiving the recombinant SCP vaccine were free of streptococci soon after infection, whereas 30-50% of the control group were culture positive for many days. Therefore, the recombinant SCP was effective as a vaccine against β-hemolytic *Streptococci.*

Also, a 2908bp fragment of the *scpA49* gene, ΔSCPA49, was ligated to the expression vector pGEX-4T-1 and expressed in *E. coli.* The purified protein induced high titers of rabbit antibodies which were able to neutralize *in vitro* peptidase activity associated with the homologous M49 streptococcal serotype, as well as the heterologous serotypes M1, M6 and M12. Intranasal immunization of mice with the ΔSCPA49 immunogen stimulated significant levels of specific salivary IgA and serum IgG antibodies and reduced the potential of wild type M1, M2, M6, M11 and M49 streptococci to colonize. Thus the SCP protein was effective as a vaccine against several serotypes of streptococci.

### Brief Description of the Drawings

Figure 1. Architecture of C5a peptidase from β-hemolytic streptococci. D indicates an aspartic acid residue; H indicates histidine; S indicates serine; L indicates leucine; P indicates proline; T indicates threonine; and N indicates asparagine. R₁, R₂, R₃ and R₄ indicate repeated sequences. The numbers indicate the amino acid residue position in the peptidase.
Figure 2. Alignment of the amino acid sequence of SCP from group A streptococci strain 49, group A streptococci strain 12 and group B streptococci (SEQ. ID. Nos. 1, 2 and 3, respectively). The sequences are identical except for the indicated amino acid positions. The triangle (∇) indicates the predicted cleavage point of the signal peptidase. Amino acids predicted to be in the enzyme's active site are marked by asterisks. Deletions in the amino acid sequence are indicated by dots and are boxed.
Figure 3. Construction of SCP insertion and deletion mutants. Black box indicates deleted region.
Figure 4. Single color FACS analysis. Fluorescence data were analyzed by gating on PMNs. A second gate was set to count high staining cells defined by the first gate. Air sacs were inoculated with 1 × 10⁶ CFU.
Figure 5. Persistence of Wild type and SCPA⁻ following intranasal infection.
Figure 6. Intranasal immunization of CD-1 mice with SPCA protein interferes with oral colonization by M type 49 streptococci.
Figure 7. Comparison of the ability of SCPA⁻ and M⁻ mutants of Group A streptococcus to colonize mice following intranasal infection. Compares BALB/c mice (ten in each experimental group) inoculated with 2 x 10⁷ CFU of M6 streptococci. Throat swabs were cultured each day on blood agar plates containing streptomycin. Mice were considered positive if plates contained one β-hemolytic colony. Data were analyzed statistically by the χ² test.
Figure 8. Construction of ΔSCPA49 vaccine and immunization protocol.
Figure 9. Serum IgG and secretory IgA responses after intranasal immunization of mice with the purified ΔSCPA49 protein. Serum and saliva levels of SCPA49 specific IgG were determined by indirect ELISA. Sera from each mouse were diluted to 1: 2,560 in PBS; saliva was diluted 1:2 in PBS.
Figure 10. Comparison of the ability of serotype M49 streptococci to colonize immunized and non-immunized CD1 female mice. Each experimental group contained 13 mice which were infected intranasally (i.n.) with 2.0 x 10⁸ CFU. The data were analyzed statistically by the χ² test. * P < 0.05, ** P< 0.01, *** P <0.001.

### Detailed Description of the Invention

An important first line of defense against infection by many bacterial pathogens is the accumulation of phagocytic polymorphonuclear leukocytes (PMNs) and mononuclear cells at the site of infection. Attraction of these cells is mediated by chemotactic stimuli, such as host factors or factors secreted by the invading organism. The C5a chemoattractant is pivotal to the stimulation of this inflammatory response in mammals. C5a is a 74 residue glycopeptide cleaved from the fifth component (C5) of complement. Phagocytic cells respond in a directed manner to a gradient of C5a and accumulate at the site of infection. C5a may be the most immediate attractant of phagocytes during inflammation. As PMNs infiltrate an inflammatory lesion they secrete other chemokines, such as IL8, which further intensify the inflammatory response.

Streptococcal C5a peptidase (SCP) is a proteolytic enzyme located on the surface of pathogenic streptococci where it destroys C5a, as C5a is locally produced. SCP specifically cleaves the C5a chemotaxin at the PMN binding site (between His⁶⁷-Lys⁶⁸ residues of C5a) and removes the seven most C-terminal residues of C5a. This cleavage of the PMN binding site eliminates the chemotactic signal. Cleary, P., et al., "Streptococcal C5a peptidase is a highly specific endopeptidase," Infect, Immun., 60:5219-5223 (1992); Wexler, D. E., et al., "Mechanism of action of the group A streptococcal C5a inactivator," Proc. Natl. Acad. Sci. USA, 82:8144-8148 (1985).

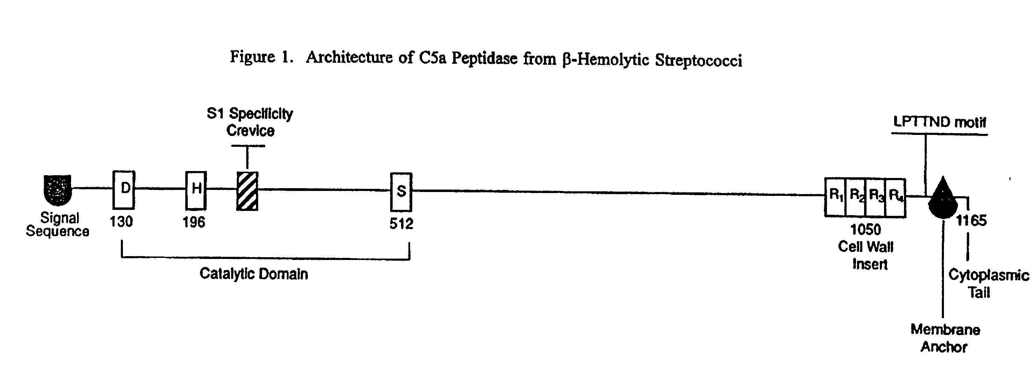

SCP from group A streptococci is a subtilisin-like serine protease with an *M*_{*r*} of 124,814 da and with a cell wall anchor motif which is common to many gram⁺ bacterial surface proteins. The architecture of C5a peptidase is given in Figure 1. The complete nucleotide sequence of the streptococcal C5a peptidase gene of *Streptococcus pyogenes* has been published. Chen, C., and Cleary, P., "Complete nucleotide sequence of the streptococcal C5a peptidase gene of Streptococcus pyogenes," J. Biol. Chem., 265:3161-3167 (1990). In contrast to subtilisins, SCP has a very narrow substrate specificity. This narrow specificity is surprising in light of the marked similarities between their catalytic domains. Cleary, P., et al., "Streptococcal C5a peptidase is a highly specific endopeptidase," Infect. Immun., 60:5219-5223 (1992). both Residues involved in charge transfer are conserved, as are residues on both sides of the binding pocket, however, the remaining amino acid sequence of SCP is unrelated to that of Subtilisins. More than 40 serotypes of Group A streptococci were found to produce SCP protein or to harbor the gene. Cleary, P., et al., "A streptococcal inactivator of chemotaxis: a new virulence factor specific to group A streptococci," in Recent Advances in Streptococci and Streptococcal Disease p.179-180 (S. Kotami and Y. Shiokawa ed.; Reedbooks Ltd., Berkshire, England; 1984); Podbielski, A., et al., "The group A streptococcal virR49 gene controls expression of four structural vir regulon genes," Infect. Immun., 63:9-20 (1995).

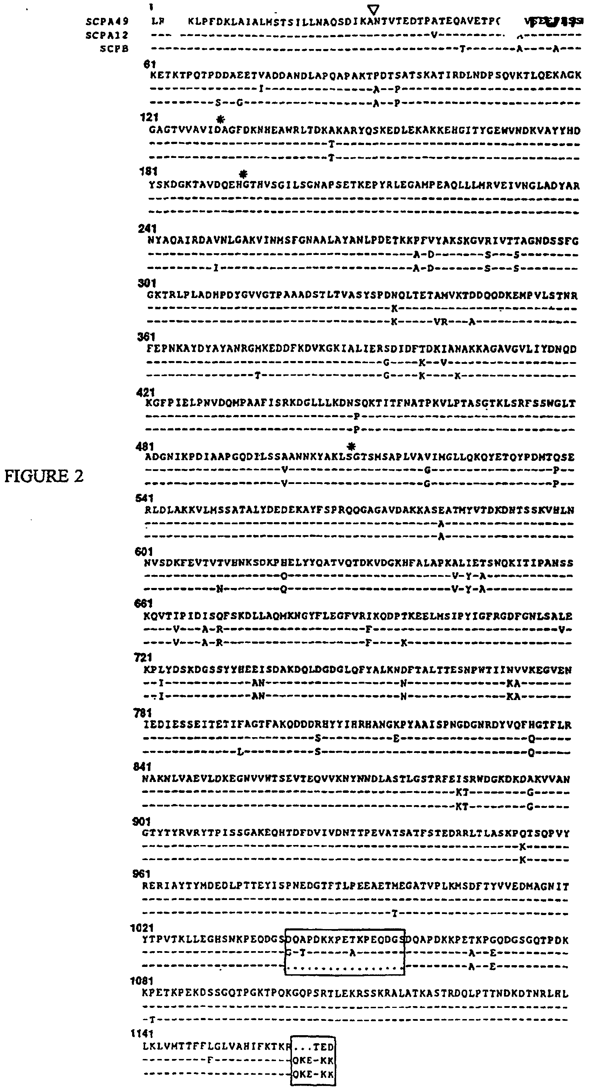

A C5a peptidase enzyme associated with group B streptococci has also been identified. Hill, H. R., et al., "Group B streptococci inhibit the chemotactic activity of the fifth component of complement," J. Immunol. 141:3551-3556 (1988). Restriction mapping and completion of the *scpB* nucleotide sequence showed that *scpB* is 97-98% similar to *scpA*. See Figure 2 for comparison of the amino acid sequence of SCP from group A streptococci strain 49, group A streptococci strain 12 and group B streptococci (SEQ. ID. Nos. 1, 2 and 3, respectively). More than 30 strains, representing all serotypes of group B streptococci carry the *scpB* gene. Cleary P.P., et al. "Similarity between the Group B and A streptococcal C5a Peptidase genes," Infect. Immun, 60:4239-4244 (1992); Suvorov A.N., et al., "C5a peptidase gene from group B streptococci," in Genetics and Molecular Biology of Streptococci, Lactococci, and Enterococci p. 230-232 (G. Dunny, P. Cleary and L McKay (ed.); American Society for Microbiology, Washington, D.C.; 1991).

Human isolates of groups G and C streptococci also harbor *scpA*-like genes. Some group G strains were shown to express C5a specific protease activity on their surface. Cleary, P. P., et al., "Virulent human strains of group G streptococci express a C5a peptidase enzyme similar to that produced by group A streptococci," Infect. Immun., 59:2305-2310 (1991). Therefore, all serotypes (>80) of group A streptococci, group B streptococci, group C streptococci and group G streptococci produce the SCP enzyme.

SCP assists streptococci to colonize a potential infection site, such as the nasopharyngeal mucosa, by inhibiting the influx of phagocytic white cells to the site of infection. This impedes the initial clearance of the streptococci by the host. The impact of SCP on inflammation, C5a leukocyte chemotaxis and streptococcal virulence was examined using streptococcal strains with well-defined mutations in the protease structural gene. SCP mutants were constructed by targeted plasmid insertion and by replacement of the wild type gene with *scpA* containing a specific internal deletion. Mutants lacked C5a protease activity and did not inhibit the chemotactic response of human or mouse PMNs to C5a in vitro.

A mouse connective tissue air sac model was used to confirm that SCP retards the influx of phagocytic cells and clearance of streptococci from the site of infection. A connective tissue air sac is generated by injecting a small amount of air and PBS (with or without streptococci in it) with a 25-gauge needle under the skin on the back of a mouse. Boyle, M.D.P. et al., "Measurement of leukocyte chemotaxis in vivo," Meth. Enzymol., 162:101:115 (1988). At the end of the experiment, the mice were euthanized by cervical dislocation, the air sacs dissected from the animals, and the air sacs homogenized in buffer. An advantage of the air sac model is that the air sac remains inflated for several days and free of inflammation, unless an irritant is injected. Thus, injected bacteria and the resulting inflammatory response remains localized over short periods of infection.

The air sac model was modified to compare clearance of wild type SCP⁺ and SCP⁻ streptococci, (i.e., group A streptococci which carried a mutant non-functional form of SCP) and to analyze the cellular infiltrate at an early stage of infection. Tissue suspensions were assayed for viable streptococci on blood agar plates and the cellular infiltrate was analyzed by fluorescent cell sorting (FACS). In FACS analysis, individual cells in suspension are labelled with specific fluorescent monoantibodies. Aliquots of labelled cells are injected into a FAC-Scan flowcytometer, or fluorescent cell sorter, which counts cells based on their unique fluorescence. The experiments using the air sac model indicated that streptococci that were SCP⁺ were more virulent than streptococci that were SCP⁻.

A study was performed to measure production of human antibody, both IgG and IgA, against SCP in human sera and saliva. O'Connor, SP, et al., "The Human Antibody Response to Streptococcal C5a Peptidase," J. Infect. Dis. 163:109-16 (1991). Generally, sera and saliva from young, uninfected children lacked antibody to SCP. In contrast, most sera and saliva specimens from healthy adults had measurable levels of anti-SCP IgG and SCP-specific secretory IgA (anti-SCP IgA). Paired acute and convalescent sera from patients with streptococcal pharyngitis possessed significantly higher levels of anti-SCP IgG than did sera from healthy individuals. Sera containing high concentrations of anti-SCP immunoglobulin were capable of neutralizing SCP activity. Detection of this antibody in >90% of the saliva specimens obtained from children who had recently experienced streptococcal pharyngitis demonstrated that children can produce an antibody response.

Even though the human subjects produced IgG and IgA against SCP in response to a natural streptococcal infection, it was not known whether the anti-SCP immunoglobulin provides any protection against infection. The basis for immunity to streptococcal infection following natural infection is poorly understood. Further, it was not known if the SCP protein could act as a vaccine against β-hemolytic streptococcal colonization or infection. First, a study was performed to examine the role of SCP in colonization of the nasopharynx. Following intranasal infection with live group A streptococci, throat cultures were taken daily for up to ten days. Wild type and isogenic SCP-deficient mutant streptococci were compared for the ability to persist in the throat over this ten day period. As predicted, the SCP-deficient mutant streptococci were cleared from the nasopharynx more rapidly.

The same intranasal mouse model was used to test the capacity of SCP to induce immunity which will prevent colonization. A mutant form of the recombinant *scpA49* gene (lacking 848-1033 nucleotides from the 5' end and 3941-4346 nucleotides from the 3' end of the gene) was cloned into and expressed from the high expression vector pGEX-4T-1. Enzymatically defective SCP protein was purified from an *E*. *coli* recombinant by affinity chromatography. Sera from rabbits vaccinated intradermally with this protein preparation neutralized SCP activity in vitro. Purified protein (40 µg) was administered intranasally to mice over a period of five weeks. Immunized mice cleared streptococci in 1-2 days; whereas, throat cultures of non-immunized mice remained positive for up to 10 days. The experiment was repeated on three sets of mice, vaccinated with three separate preparations of a SCP protein.

Further experiments were performed to determine whether immunization of an animal with a streptococcal C5a peptidase from one group A serotype would prevent colonization by heterologous serotypes. A 2908 bp fragment of the *scpA49* gene was cloned into an expression vector and expressed in *E. coli.* The affinity purified ΔSCPA49 protein proved to be highly immunogenic in mice and rabbits. Although the purified ΔSCPA49 immunogen lacked enzymatic activity, it induced high titers of rabbit antibodies that were able to neutralize *in vitro* peptidase activity associated with M1, M6, M12 and M49 streptococci. This confirmed that anti-peptidase antibodies have cross-neutralizing antibody activity. Four sets of mice were then intranasally immunized with ΔSCPA49 and each was challenged with a different serotype of group A streptococcus. The immunization of mice with the deleted form of the SCPA49 protein stimulated significant levels of specific salivary IgA and serum IgG antibodies and reduced the potential of wild type M1, M2, M6, M11 and M49 streptococci to colonize. These experiments confirm that immunization with streptococcal C5a peptidase vaccine is effective in preventing the colonization of the nasopharynx.

The present invention thus provides a vaccine for use to protect mammals against β-hemolytic *Streptococcus* colonization or infection. In one embodiment of this invention, as is customary for vaccines, the enzymatically inactive streptococcal C5a peptidase, variant or fragment thereof, can be delivered to a mammal in a pharmacologically acceptable vehicle. As one skilled in the art will appreciate, it is not necessary to use the entire protein. A selected portion of the polypeptide (for example, a synthetic immunogenic polypeptide corresponding to a portion of the streptococcal C5a peptidase) can be used.

As one skilled in the art will also appreciate, it is not necessary to use a polypeptide that is identical to the native SCP amino acid sequence. The amino acid sequence of the immunogenic polypeptide can correspond essentially to the native SCP amino acid sequence. As used herein "correspond essentially to" refers to a polypeptide sequence that will elicit a protective immunological response at least substantially equivalent to the response generated by native SCP. An immunological response to a composition or vaccine is the development in the host of a cellular and/or antibody-mediated immune response to the polypeptide or vaccine of interest. Usually, such a response consists of the subject producing antibodies, B cell, helper T cells, suppressor T cells, and/or cytotoxic T cells directed specifically to an antigen or antigens included in the composition or vaccine of interest. Vaccines of the present invention can also include effective amounts of immunological adjuvants, known to enhance an immune response.

Alternatively, the SCP of the invention can be conjugated or linked to another peptide or to a polysaccharide. For example, immunogenic proteins well-known in the art, also known as "carriers," may be employed. Useful immunogenic proteins include keyhole limpet hemocyanin (KLH), bovine serum albumin (BSA), ovalbumin, human serum albumin, human gamma globulin, chicken immunoglobulin G and bovine gamma globulin. Useful immunogenic polysaccharides include group A *Streptococci* polysaccharide, C-polysaccharide from group B *Streptococci,* or the capsular polysaccharide of *Streptococci pnuemoniae*. Alternatively, polysaccharides of other pathogens that are used as vaccines can be conjugated or linked to SCP.

To immunize a subject, the SCP of the invention or an immunologically active fragment or mutant thereof, is administered parenterally, usually by intramuscular or subcutaneous injection in an appropriate vehicle. Other modes of administration, however, such as oral delivery or intranasal delivery, are also acceptable. Vaccine formulations will contain an effective amount of the active ingredient in a vehicle, the effective amount being readily determined by one skilled in the art. The active ingredient may typically range from about 1% to about 95% (w/w) of the composition, or even higher or lower if appropriate. The quantity to be administered depends upon factors such as the age, weight and physical condition of the animal or the human subject considered for vaccination. The quantity also depends upon the capacity of the animal's immune system to synthesize antibodies, and the degree of protection desired. Effective dosages can be readily established by one of ordinary skill in the art through routine trials establishing dose response curves. The subject is immunized by administration of the SCP of the invention or fragment thereof in one or more doses. Multiple doses may be administered as is required to maintain a state of immunity to streptococci.

Intranasal formulations may include vehicles that neither cause irritation to the nasal mucosa nor significantly disturb ciliary function. Diluents such as water, aqueous saline or other known substances can be employed with the subject invention. The nasal formulations may also contain preservatives such as, but not limited to, chlorobutanol and benzalkonium chloride. A surfactant may be present to enhance absorption of the subject proteins by the nasal mucosa.

Oral liquid preparations may be in the form of, for example, aqueous or oily suspension, solutions, emulsions, syrups or elixirs, or may be presented dry in tablet form or a product for reconstitution with water or other suitable vehicle before use. Such liquid preparations may contain conventional additives such as suspending agents, emulsifying agents, nonaqueous vehicles (which may include edible oils), or preservative.

To prepare a vaccine, the purified SCP, subunit or mutant thereof of the invention, car be isolated, lyophilized and stabilized. The SCP peptide may then be adjusted to an appropriate concentration, optionally combined with a suitable vaccine adjuvant, and packaged for use. Suitable adjuvants include but are not limited to surfactants, e.g., hexadecylamine, octadecylamine, lysolecithin, dimethyldioctadecylammonium bromide, N,N-dioctadecyl-N'-N-bis(2-hydroxyethyl-propane di-amine), methoxyhexadecyl-glycerol, and pluronic polyols; polanions, e.g., pyran, dextran sulfate, poly IC, polyacrylic acid, carbopol; peptides, e.g., muramyl dipeptide, aimethylglycine, tuftsin, oil emulsions, alum, and mixtures thereof. Other potential adjuvants include the B peptide subunits of *E*. *coli* heat labile toxin or of the cholera toxin. McGhee, J.R., et al., "On vaccine development," Sem. Hematol., 30:3-15 (1993). Finally, the immunogenic product may be incorporated into liposomes for use in a vaccine formulation, or may be conjugated to proteins such as keyhole limpet hemocyanin (KLH) or human serum albumin (HSA) or other polymers.

The application of SCP, subunit or mutant thereof of the invention, for vaccination of a mammal against colonization offers advantages over other vaccine candidates. Prevention of colonization or infection by inoculation with a single protein will not only reduce the incidence of the very common problems of strep throat and impetigo, but will also eliminate sequelae such as rheumatic fever, acute glomerulonephritis, sepsis, toxic shock and necrotizing fascitis.

The following examples are intended to illustrate but not limit the invention.

### EXAMPLE 1

### Construction of insertion and deletion mutants in scpA49 and scpA6

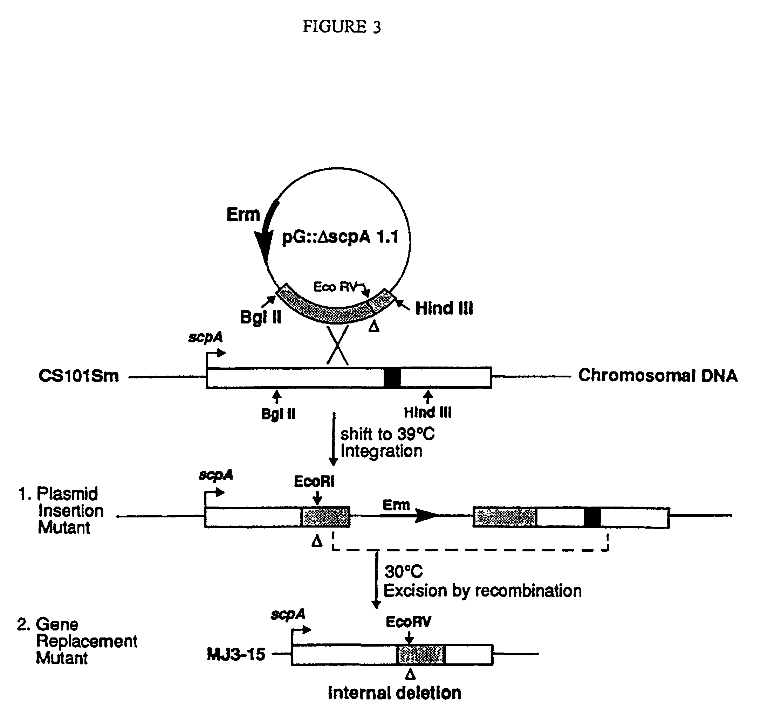

**a) Bacterial strains and culture conditions.** *S. pyogenes* strain CS101 is a serotype M49, and OF⁺ strain. CS159 is a clinical isolate with a deletion which extends through the M gene cluster and *scpA*. A spontaneous, streptomycin resistant derivative of strain CS101, named CS101Sm, was selected by plating streptococci from a stationary phase culture on tryptose blood agar containing streptomycin (200 µg/ml). CS 101::pG⁺host5 is strain CS101 with pG⁺host5 integrated into the chromosome at an unknown location, but outside *scpA* and the *emm* gene cluster. *Escherichia coli* strain ER1821 (from New England Biolabs, Inc. Beverly, MA) was used as the recipient for the suicide vector, plasmid pG⁺host5. Plasmid pG⁺host5 was obtained from Appligene, Inc. Pleasanton, CA. Streptococci were grown in Todd-Hewitt broth supplemented with 2% neopeptone or 1% yeast extract, or on tryptose agar plates with 5% sheep blood. *E. coli* strain ER1821 containing plasmid pG⁺host5 was grown in LB broth with erythromycin (300 µg/ml). Streptococci with plasmid pG⁺host5 were cultured in Todd-Hewitt broth with 1% yeast extract (THY) containing 1 µg/ml of erythromycin (Erm).
   SCP refers to streptococcal C5a peptidase from β-hemolytic *Streptococcus* generally. SCPA12, SCPA49, SCPA6 are the specific peptidases from group A *Streptococcus* M type 12, 49 and 6 strains, respectively. The term *scpA* refers to the gene encoding SCP from group A streptococci. *ScpA12*, *scpA6* and *scpA49* are the genes encoding the SCPA12, SCPA49 and SCPA6 peptidases. SCPB and *scpB* refer to the peptidase and gene from group B streptococci. The amino acid sequences for SCPA49 (SEQ. ID. No. 1), SCPA12 (SEQ. ID. No. 2) and SCPB (SEQ. ID. No. 3) are given in Figure 2.
**b) Construction of *scpA* insertion mutant.** Well-defined insertion mutants of *scpA* were constructed using plasmid insertion and gene replacement methods. An internal *scpA49 BgIII - BamHI* fragment, the insertion target, was ligated into the thermosensitive shuttle vector pG⁺host5 to form plasmid pG::scpA1.2 and transformed into *E*. *coli* ER1821 (Fig. 3). The pG⁺host5 vector contains an *E*. *coli* origin of replication that is active at 39°C, a temperature sensitive Gram⁺ origin of replication (active at 30°C and inactive at 39°C in streptococci), and an erythromycin resistance gene for selection. High temperature forces the plasmid to integrate into the chromosomal DNA of group A streptococci by homologous recombinant at frequencies ranging from 10⁻² to 10⁻³.
   Recombinant plasmid DNA pG::scpA1.2 was electroporated into CS101 recipient cells. Transformants were selected on THY-agar plates containing 1 µg/ml erythromycin at 30°C. Chromosomal integrants which resulted from recombination between the plasmid insert and the chromosomal *scpA* were selected by erythromycin resistance at 39°C. Two insertion mutants, M14 and M16, were analyzed. EmrS revertants of strain M14 and M16 were obtained by passage in THY without antibiotic at 30°C and finally plated at 37°C without Erm selection. Colonies that had lost the plasmid were isolated to confirm that the mutant phenotype resulted from insertion of the plasmid into *scpA49*, rather than from a simultaneous unrelated mutation.
**c) Introduction of a defined deletion into *scpA*.** A mutant strain with a defined deletion internal to *scpA* was constructed to eliminate the possibility that insertions in *scpA* could be polar and reduce expression of downstream genes, unknown genes which could also contribute to the organism's virulence. First, a defined deletion in *Bgl*II-*Hind*III fragment of *scpA* was produced by inside-out PCR with primer 1 (5'-GGGGGG**GAATTC**GTAGCGGGTATCATGGGAC-3'), SEQ. ID. No. 4, and primer 2 (5'-GGGGGG**GAATTC**GGGTGCTGCAATATC- TGGC-3'), SEQ. ID No. 5. Underlined nucleotides correspond to *scpA* sequences with coordinates 2398 and 2322, respectively, and the bold faced nucleotides correspond to a *EcoR*I recognition site. The primers were selected to produce an in-frame deletion in the *scpA* gene. These primers copy plasmid DNA in opposite directions and define the boundaries of the deletion. Innis, M.A., et al., eds., PCR Protocols A Guide to Methods and Applications (Academic Press, 1990). Plasmid pG::scpA1.2 DNA was used as template.
   The amplified product was digested with *EcoR*I and ligated to plasmid pG⁺host5. The resulting plasmid pG::ΔscpA1.1 contained an 76 bp deletion internal to *scpA*. This in-frame deletion removed 25 amino acids, including the serine which forms part of the predicted catalytic center of serine proteases. Chen, C., and Cleary, P., "Complete nucleotide sequence of the streptococcal C5a peptidase gene of Streptococcus pyogenes," J. Biol, Chem., 265:3161-3167 (1990). An EcoRV site was created at the point of deletion. DNA which overlaps the deletion was sequenced to confirm the boundaries of the deletion.
   The plasmid pG::*scpA*1.1, which contains the deletion, was transformed into *E*. *coli* ER1821. Colonies were selected for ErmR and then screened for the appropriate *scpA* deletion using miniprep plasmid DNA restricted by *EcoR*I. The precise boundaries of the deletion were confirmed by DNA sequencing. Plasmid pG::ΔscpA1.1 was electroporated into strain CS101Sm as described above, then integrants were selected by grown on Erm at 39°C. Integration of the plasmid into the chromosome of the M49 strain CS101sm using high temperature selection. The insertion location was confirmed by PCR. Growth of CS101Sm (pG::*scpA*1.1) at low temperature without erythromycin selection resulted in high frequency segregation of ErmS revertants which have lost the plasmid by random deletion event or by excision due to recombination between the duplicated *scpA* sequences created by the insertion. Two deletion mutants were identified, MJ2-5 and MJ3-15, and were studied further. The chromosomal deletion left behind by recombinational excision of plasmid pG::*scpA*1.1 was defined by PCR and Southern hybridization to EcoRV digested DNA.
**d) In vitro effects on SCP.** The impact of insertions and deletions on the expression of SCP antigen and peptidase activity was assessed by Western blot and PMNs adherence assays. Streptococci were incubated in 100 ml THY at 37°C overnight. The culture pellet was washed two times in 5 ml cold 0.2 M NaAcetate (pH 5.2), then suspended in 1 ml TE-sucrose buffer (20% sucrose 10 mM Tris, 1 mM EDTA, pH 7.0) and 40 µl Mutanolysin. The mixture was rotated at 37°C for 2 hr, then centrifuged 5 min at 4500 rpm. Supernatants contained protease inhibitor, 100 mM phenylmethyl sulfonyl fluoride (PMSF). Electrophoresis and Western blotting methods were performed as described in Laemmli, U. K., "Cleavage of structural proteins during the assembly of the head of bacteriophage T4," Nature 227:680-685 (1970). For colony blots, colonies were grown on THY-agar plates, printed onto nitrocellulose membrane (BioBlot-Nc, Costor, Cambridge, MA), fixed under an infrared lamp for 10 min. and exposed to antibody. O'Connor, S. P. and Cleary, P. P., "In vivo Streptococcus pyogenes C5a peptidase activity," J. Infect. Dis. 156:495-506 (1987). The primary antiserum used to detect SCP protein on Western and colony blots was prepared by immunization of a rabbit with purified recombinant SCP protein. Binding was detected by anti-rabbit antibody alkaline phosphatase conjugate.
   C5a peptidase activity was measured using a PMN adherence assay. Booth, S. A. et al., "Dapsone suppresses integrin-mediated neutrophil adherence function," J. Invest. Dermatol, 98:135-140 (1992). After incubation of C5a (Sigma, St. Louis, MO) with streptococcal extracts or purified protease, residual C5a can activate PMNs to become adherent to BSA coated wells. First, microtiter wells were coated with 0.5% BSA in PBS and incubated for 1 hr at 37°C. Human PMNs were isolated by centrifugation in Ficoll Hypaque (Sigma, St. Louis, MO). 40 µl of intact streptococci or protein extracts were incubated with 20 µl of 5 µM C5a in 340 µl of PBS with 1% glucose and 0.1% CaCl₂ at 37°C for 45 min. BSA-coated wells were washed with PBS, and resuspended PMNs and residual C5a were added to wells. The mixture was incubated for 45 min at 37°C in 7% CO₂. Finally, wells were washed to remove nonadherent PMNs. Adherent PMNs were stained with crystal violet and the OD₅₇₀ₙₘ was read in an ELISA reader. The optical density is proportional to the amount of residual C5a or inversely proportional to the amount of SCP activity.
   Insertion mutants completely lacked SCPA49 antigen; whereas, deletion mutants MJ2-5 and MJ3-15, as expected produced SCP antigen. Both whole cells and mutanolysin protein extracts from M14, M16, M2-5 and MJ3-15 lacked the ability to destroy rC5a activated adherence of PMNs to microtiter plates. A small amount of residual inhibitory activity (10-15%) associated with mutant extracts may be due to toxic effects of the extract on the neutrophils.

### EXAMPLE 2

### SCP delays Recruitment of Phagocytes and Clearance of Streptococci from Subdermal Sites of Infection

In order to verify that SCP was responsible for the inactivation of C5a, the insertion and deletion mutants of *scpA* were constructed as described in Example 1 above, and tested for activity. When insertions or deletions were introduced into *scpA*, the mutant SCP was not able to destroy C5a-activated adherence of PMNs to microtiter plates.

The impact of mutations in *scpA* on virulence was tested using an animal model where streptococci remained localized, and where the influx of inflammatory cells could be analyzed. To test the hypothesis that SCP functions very early to retard initial clearance of the organism, the fate of SCP⁺ and SCP⁻ streptococci just 4 hours after inoculation of connective tissue air sacs was compared. Moreover, the dissemination of streptococci to lymph nodes and spleens after this short period of infection was also assessed. CD1 male outbred mice (25 g) obtained from Charles River Breeding Laboratory, Wilmington, MA were used for all experiments. A connective tissue air sac was generated by injecting 0.9 ml of air and 0.1 ml group A streptococci diluted in PBS with a 25-gauge needle under the skin on the back of the mouse. In some experiments the SCP⁺ CS101::pG⁺host5 was used as a positive control. In other experiments strain CS101Sm was used as the positive control. Mice were euthanized by cervical dislocation 4 hours after infection. Where indicated all four inguinal lymph nodes, spleen and air sac were dissected from the animals and homogenized in PBS. Tissue suspensions were assayed for viable colony forming unit (CFU) on blood agar plates containing 1 µg/ml erythromycin or 200 µg/ml streptomycin.

In a preliminary experiment air sacs were fixed on slides, stained with Wright's stain and examined microscopically. Although counts of granulocytes by this method were unreliable, there appeared to be significantly fewer residual SCP⁻ than wild type streptococci in fixed tissue. Additional experiments were performed in an attempt to measure this difference. Dispersed cell populations of air sacs were prepared by grinding the air sac in PBS and passing them through Nylon monofilament mesh (TETKO Co. New York).

The cells were pelleted by centrifugation 5 min at 300 × g and resuspended at 5 × 10⁶/ml in FACS buffer (Hank's balanced salt solution without phenol red, 0.1% NaN₃, 1.0% BSA fraction V). Cells (1.0 × 10⁶) were stained directly with 1 µg FITC anti-mouse Mac-1 or indirectly with 1 µg Biotin conjugated anti-mouse Gr-1 followed by 1 µg Streptavidin labelled with fluorescene or FITC. Monoclonal antibodies, Mac-1 and Gr-1, were obtained from Pharmingen, Inc. CA. Labeled cells were fixed in 1.0% paraformaldehyde. Fluorescence profiles were generated using a FAC-Scan flowcytometer and Consort 32 software (Becton Dickinson). Mouse PMNs were purified from whole blood by Ficoll Hypaque density gradient centrifugation and used as a standard to defined PMNs in mixed populations. For measurement of specifically labeled cells, the mean fluorescence for each antibody marker was determined and gates were set to reflect intensely labeled cells. Controls included unstained cells, and cells exposed to only streptavidin FITC.

Two experiments were performed. The first compared the *scpA49* insertion mutant M16 to its SCP⁺ parent culture, strain CS101. The second compared the *scpA49* deletion mutant MJ3-15, to its parent, strain CS101Sm. (Table 1) In both experiments homogenized air sacs from mice inoculated with SCP⁻ streptococci contained fewer numbers of streptococci after 4 hours than air sacs inoculated with wild type streptococci. The first experiment showed a two-fold reduction and the second showed a four-fold reduction. These differences were statistically significant at P<0.05 and P<0.001, respectively, using an Unpaired t-test. It was also observed that wild type SCP⁺ streptococci were found in spleen homogenates from 7 of 8 mice and 6 of 8 mice; whereas, the SCP⁻ mutants were rarely found in the spleen. The opposite was true for lymph node homogenates. Nodes from 10 of 16 mice infected with SCP⁻ streptococci harbored viable streptococci; whereas, only 4 of 16 nodes from mice infected with wild type streptococci contained viable bacteria. This difference was determined to be statistically significant at P<0.05 using the Fisher's exact test.

**Table 1:**

| **Distribution of SCP**^{**+**} **and SCP**^{**-**} **streptococci 4 hours after air sac infection** | | | | |
|---|---|---|---|---|
| Strains | No. of Mice^{a} | No. of positive cultures | | Homogenized Air Sac^{c} |
| | | spleen^{b} | lymph node | |
| CS101pG (SCP⁺) | 8 | 7 | 2 | 1.3 × 10⁸ ± 2.2 × 10⁷ |
| M16 (SCP⁻) | 8 | 0 | 5 | 6.0 × 10⁷ ± 1.3 ×10⁷ |
| CS101Sm (SCP⁺) | 8 | 6 | 2 | 1.6 × 10⁸ ± 2.6 × 10⁷ |
| MJ3-15 (SCP⁻) | 8 | 1 | 5 | 3.7 × 10⁷ ± 1.5 × 107 |

| | | | | |
|---|---|---|---|---|
| ^{a} Each mouse was inoculated with 3 × 10⁸ CFU of stationary phase streptococci. | | | | |
| ^{b} Difference in the frequency of isolation of SCP⁺ streptococci from spleens relative to SCP⁻ streptococci was statistically significant (P < 0.05) for each experiment by the Fisher's exact test. | | | | |
| ^{c} Differences in CFU isolated from homogenized air sacs (means ± SEMs) were significant, strains CS101pG (SCP⁺) and M16 (SCP⁻) and MJ3-15 (SCP⁻) (P < 0.001) for each experiment by unpaired t test. | | | | |

The more rapid clearance of streptococci from air sacs resulted from more intense recruitment of PMNs. The total cell population, the percentage of Mac-1 positive granulocytes (Springer, G. et al., "Mac-1:macrophage differentiation antigen identified by monoclonal antibody," Eur. J. Immunol. 9:301-306 (1979)), and the percentage of Gr-1 positive PMN (Brummer, E. et al., "Immunological activation of polymorphonuclear neutrophils for fungal killing: studies with murine cells and blastomyces dermatitidis in vitro," J. Leuko. Bio. 36:505-520 (1984)) in air sacs were compared by single color FACS analysis. Clark, J. M., "A new method for quantitation of cell-mediated immunity in the mouse," J. Reticuloendothel. Soc. 25:255-267 (1979). Briefly, in a FACS analysis, individual cells in suspension are labelled with specific fluorescent monoantibodies. Aliquots of labelled cells are injected into a FAC-Scan flowcytometer or fluorescent cell sorter which counts cells based on their unique fluorescence.

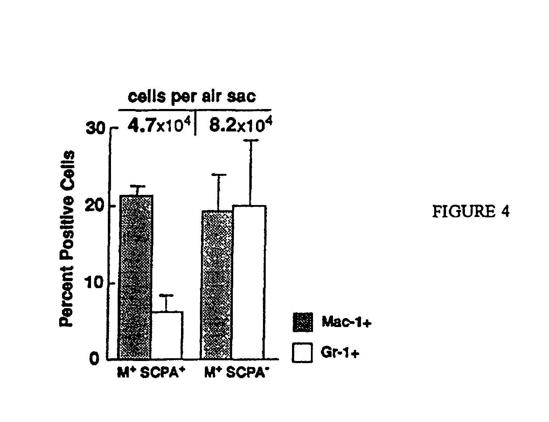

Air sacs infected with the SCP⁻ deletion mutant contained twice as many inflammatory cells as those inoculated with SCP⁺ streptococci (Fig. 4). A hundred-fold increase in the inoculum size did not alter this difference. Air sacs infected with 1 × 10⁶ SCP⁻ cells, strain MJ3-15, contained three times more Gr-1 positive cells than those inoculated with the SCP⁺ culture. In airs sacs inoculated with SCP⁺ streptococci approximately 6% of the cells were PMNs and 21% were other kinds of Mac-1⁺ granulocytes, including PMNs. In contrast, air sacs inoculated with SCP⁻ streptococci contained predominately PMNs. Gr-1 positive cells were equal to or greater than the number of Mac-1 positive cells. Flow cytometer gates were set to measure only high staining granulocytes. The remaining 70-80% of cells not stained with either antibody were likely either low staining granulocytes, red blood cells or lymphocytes. Large numbers of lymphocytes were observed microscopically in Wrights stained air sac preparations.

SCP⁺ colonies of streptococci that emerged from spleen homogenates were highly encapsulated, resembling water drops. In contrast the few SCP⁻ colonies arising from lymph nodes, were more like the inoculum. They were mixtures of non-mucoid and moderately mucoid colonies. These data suggest that M⁺SCP⁺ encapsulated streptococci can adapt, multiply and invade the bloodstream within 4 hours after infection. The basis for differential trafficking of mutant and wild type streptococci may be due to the more vigorous influx of phagocytic cells in response to SCP⁻ bacteria. Macrophages and/or skin dendritic cells may more rapidly engulfed SCP streptococci and delivered them to lymph nodes. Reduction of mutant streptococci relative to wild type is an unexpected finding, because SCP⁻ streptococci are M⁺ and resistant to phagocytosis by human neutrophils in vitro.

### EXAMPLE 3

### SCP is required for colonization of the mouse nasopharynx

Mice were inoculated intranasally to evaluate the relative capacity of wild type (SCP⁺) and SCP⁻ streptococci to colonize the nasopharynx. Streptomycin resistant M49 strain CS101 and deletion mutant MJ3-15 were used in these experiments. Cultures were not mouse passed in order to avoid selection of variants that might be uniquely mouse virulent, but no longer depend on M protein and/or SCP for persistence in the animal.

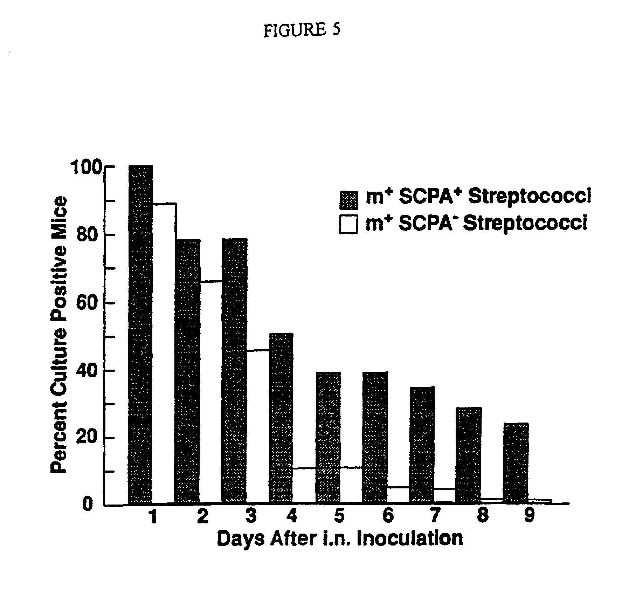

CD1 outbred mice were intranasally inoculated with 2 × 10⁸ stationary phase CFU. The nasopharynxes of anesthetized mice were swabbed daily for 8-10 days and streaked on blood agar containing streptomycin. Differences between SCP⁺ and SCP⁻ were evident by day 1, however, statistically significant differences were not observed until days 3 and 4 (Fig. 5). By day four 9/18 mice infected with M⁺SCP⁺ streptococci produced positive throat cultures, whereas only 2/18 mice infected with M⁺SCP⁻ strain retained streptococci in their throats. Four of 18 mice died from infection with SCP⁺ streptococci. None of the mice following infection with SCP⁻ bacteria succumbed to the infection. The numbers of colonies on the blood agar plates were also consistent with more rapid clearance of SCP⁻ streptococci. For example, on the third day cultures from seven mice contained >100 SCP⁺ CFU, whereas, only one mouse inoculated SCP⁻ streptococci contained > 100 CFU.

Because M49 streptococci are more often associated with skin infections the above experiments were repeated with an M6 strain, a serotype more often associated with throat infections. An insertion mutant, strain AK1.4, was constructed using the M6 strain UAB200 and the strategy previously described in Example 1. Strain AK1.4 was also cleared more rapidly than the wild type M6 culture from the nasopharynx. The above experiments confirm that group A streptococci are dependent upon SCP for persistence in the mouse nasopharynx. All SCP⁻ mutants used in the above experiments were M⁺, i.e. they resisted phagocytosis by fresh human blood. Yet, they were cleared from the nasopharyngeal mucosa.

### EXAMPLE 4

### Intranasal immunization of mice with purified recombinant SCPA49 blocks colonization following intranasal challenge

A PCR fragment which corresponds to a deleted form of the *scpA*49 gene was cloned from CS 101 M49 group A streptococci. This fragment was amplified by PCR using a forward primer beginning at nucleotide 1033 and a reverse primer beginning at nucleotide 3941 (numbering corresponding to that of Chen, C., and Cleary, P., "Complete nucleotide sequence of the streptococcal C5a peptidase gene of Streptococcus pyogenes," J. Biol. Chem., 265:3161-3167 (1990)). The fragment was ligated to the thrombin binding site of glutathione transferase gene on the pGEX-4T-1 high expression vector from Pharmacia Inc. The plasmid containing *scpA* designated pJC6 has been deposited in the American Type Culture Collection, 12301 Parklawn Drive, Rockville, MD, 20852, USA under the provision of the Budapest Treaty on October 15, 1996, and assigned ATCC accession number 98225.

The transferase-SCP fusion protein from one *E*. *coli* clone was expressed and purified by affinity chromatography on a glutathione Sepharose 4b column. All methods are described by the manufacturer (Pharmacia). The dSCP was cleaved from the hybrid protein by thrombin digestion. The thrombin was removed from eluted SCP by chromatography on a benzamidine Sepharose 6B column (Pharmacia). The affinity purified protein was confirmed to be pure SCPA49 by SDS-PAGE and by Western blot. Hyperimmune antiserum, directed against purified SCPA49 was prepared in rabbits. The recombinant SCP was not functional as a peptidase.

Two groups of mice were immunized by administration of 10 µl into each nostril, a total of 40 µg of protein, four times over a period of five weeks. Control mice received only PBS. Prior to infection sera pooled from groups of 5 mice were determined by ELISA to have high titers of anti-SCPA49 antibody. See Table 2.

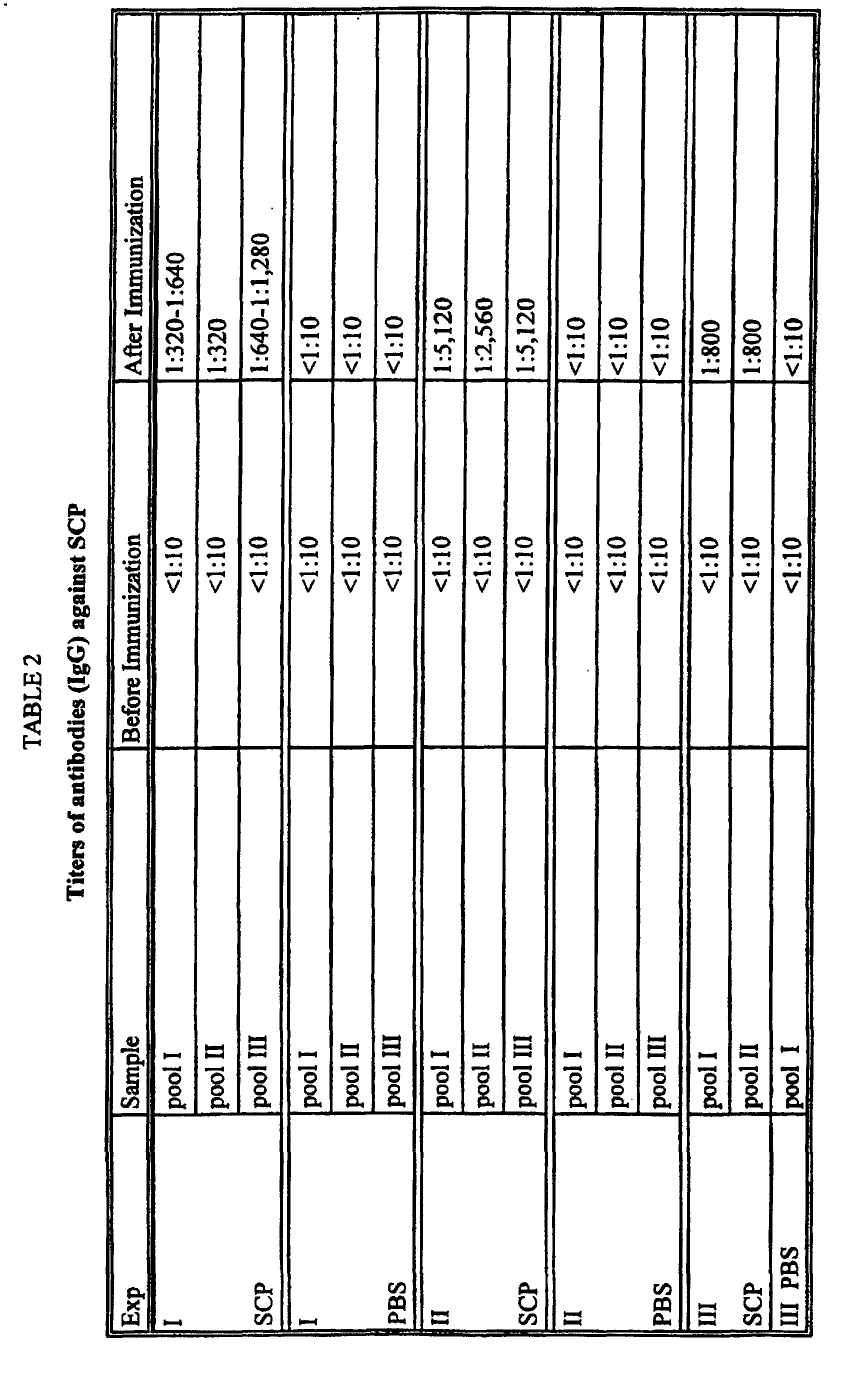

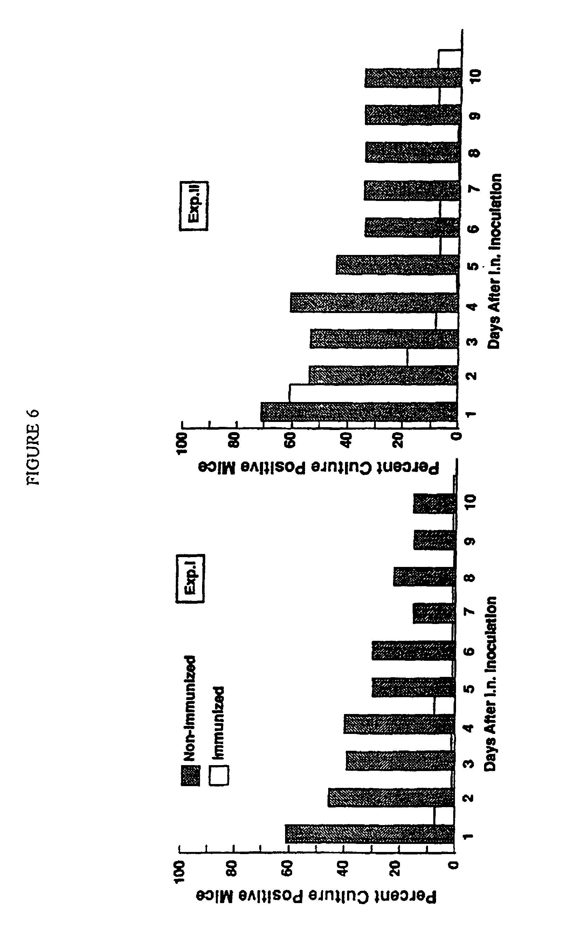

Mice were challenged with 3 × 10⁸ CFU of the wild type, CS101sm strain, 7 days after the last vaccine booster. In two separate experiments immunized mice were free of streptococci 48 hrs after infection (Fig. 6; Tables 3 and 4). In contrast 30-50% of non-vaccinated controls remained culture positive for six days, and some were still positive ten days after infection. Differences were determined to be statistically significant by the Fisher exact test. Infection of a third group of immunized and control mice produced similar results.

High titer rabbit serum directed against this mutant SCPA49 protein was able to neutralize peptidase activity associated with intact M1, M12, and M6 streptococci in vitro, confirming that peptidase lacks serotype specificity. Therefore, even SCP which is not functional as a peptidase is effective as a vaccine. It should be noted that pre-incubation of M49 streptococci with rabbit anti-SCP prior to i.n. inoculation of mice did not reduce colonization.

**Table 3:**

| **Throat cultures for streptococci after intranasal challenge of mice vaccinated intranasally with PBS or SCP expressed in *E*. *coli* DH5α (CFU after vaccine)** | | | | | | | | | | |
|---|---|---|---|---|---|---|---|---|---|---|
| Days after challenge | | | | | | | | | | |
| Mice | 1 | 2 | 3 | 4 | 5 | 6 | 7 | 8 | 9 | 10 |
| PBSCT-II | | | | | | | | | | |
| 1 | 0 | 0 | 0 | 0 | 0 | 0 | 0 | 0 | 0 | 0 |
| 2 | 3 | 0 | 0 | 0 | 0 | 0 | 0 | 0 | 0 | 0 |
| 3 | 77 | >200 | 150 | 4 | 11 | 3 | 0 | 51 | 97 | 53 |
| 4 | 9 | >200 | >200 | 3 | 11 | 3 | 0 | 0 | 0 | 0 |
| 5 | 0 | 0 | 0 | 0 | 0 | 0 | 0 | 0 | 0 | 0 |
| 6 | 4 | 6 | 45 | 47 | 3 | >200 | 29 | >200 | 83 | 70 |
| 7 | 15 | 194 | >200 | 9 | 172 | 10 | 5 | 3 | 0 | 0 |
| 8 | 0 | 0 | 0 | 0 | 0 | 0 | 0 | 0 | 0 | 0 |
| 9 | 0 | 32 | 4 | | 4 | 0 | 0 | 0 | 0 | 0 |
| 10 | 2 | 0 | 0 | 0 | 0 | 0 | 0 | 0 | 0 | 0 |
| 11 | 3 | 0 | 0 | 0 | 0 | 0 | 0 | 0 | 0 | 0 |
| 12 | 0 | 0 | 0 | 0 | 0 | 0 | 0 | 0 | 0 | 0 |
| 13 | 127 | 4 | 0 | 0 | 0 | 0 | 0 | 0 | 0 | 0 |
| No. of positive | 8 | 6 | 5 | 5 | 4 | 4 | 2 | 3 | 2 | 2 |

| SCPAD-II | | | | | | | | | | |
|---|---|---|---|---|---|---|---|---|---|---|
| 1 | 0 | 0 | 0 | 0 | 0 | 0 | 0 | 0 | 0 | 0 |
| 2 | 0 | 0 | 0 | 0 | 0 | 0 | 0 | 0 | 0 | 0 |
| 3 | 0 | 0 | 0 | 0 | 0 | 0 | 0 | 0 | 0 | 0 |
| 4 | 0 | 0 | 0 | 0 | 0 | 0 | 0 | 0 | 0 | 0 |
| 5 | 35 | 0 | 0 | 0 | 0 | 0 | 0 | 0 | 0 | 0 |
| 6 | 0 | 0 | 0 | 0 | 0 | 0 | 0 | 0 | 0 | 0 |
| 7 | 0 | 0 | 0 | 0 | 0 | 0 | 0 | 0 | 0 | 0 |
| 8 | 0 | 0 | 0 | 0 | 0 | 0 | 0 | 0 | 0 | 0 |
| 9 | 0 | 0 | 0 | 0 | 0 | 0 | 0 | 0 | 0 | 0 |
| 10 | 0 | 0 | 0 | 0 | 0 | 0 | 0 | 0 | 0 | 0 |
| 11 | 0 | 0 | 0 | 21 | 0 | 0 | 0 | 0 | 0 | 0 |
| 12 | 0 | 0 | 0 | 0 | 0 | 0 | 0 | 0 | 0 | 0 |
| 13 | 0 | 0 | 0 | 0 | 0 | 0 | 0 | 0 | 0 | 0 |
| No. of positive | 1 | 0 | 0 | 1 | 0 | 0 | 0 | 0 | 0 | 0 |

**Table 4:**

| **Throat cultures for streptococci after intranasal challenge of mice vaccinated intranasally with PBS or SCP expressed in *E*. *coli* DH5α (CFU after vaccine**) | | | | | | | | | | |
|---|---|---|---|---|---|---|---|---|---|---|
| Days after challenge | | | | | | | | | | |
| Mice>* | 1 | 2 | 3 | 4 | 5 | 6 | 7 | 8 | 9 | 10 |
| PBSCT-I | | | | | | | | | | |
| 1 | 112 | 143 | 85 | 16 | 0 | 0 | 0 | 0 | 0 | 0 |
| 2 | 127 | 27 | 18 | 89 | 3 | 7 | 7 | 7 | 70 | 3 |
| 3 | >200 | >200 | >200 | >200 | >200 | >200 | >200 | 108 | >200 | 66 |
| 4 | 31 | 200 | 4 | 2 | 0 | 0 | 0 | 0 | 0 | 0 |
| 5 | 4 | 0 | 0 | 3 | 3 | 0 | 0 | 0 | 0 | 0 |
| 6 | 0 | 0 | 0 | 0 | 0 | 0 | 0 | 0 | 0 | 0 |
| 7 | >200 | >200 | 120 | 125 | 91 | 145 | >200 | >200 | >200 | 166 |
| 8 | 2 | 0 | 0 | 0 | 0 | 0 | 0 | 0 | 0 | 0 |
| 9 | 0 | 0 | 0 | 0 | 0 | 0 | 0 | 0 | 0 | 0 |
| 10 | 0 | 0 | 0 | 0 | 0 | 0 | 0 | 0 | 0 | 0 |
| 11 | 37 | >200 | 194 | 16 | >200 | 47 | >200 | 101 | >200 | >200 |
| No. of positive | 8 | 6 | 6 | 7 | 5 | 4 | 4 | 4 | 4 | 4 |

| SCPAD-I | | | | | | | | | | |
|---|---|---|---|---|---|---|---|---|---|---|
| 1 | 6 | 0 | 0 | 0 | 0 | 0 | 0 | 0 | 0 | 0 |
| 2 | 105 | 41 | 0 | 0 | 0 | 0 | 0 | 0 | 0 | 0 |
| 3 | 0 | 0 | 0 | 0 | 0 | 0 | 0 | 0 | 0 | 0 |
| 4 | 2 | 0 | 0 | 0 | 0 | 0 | 0 | 0 | 0 | 0 |
| 5 | 2 | 0 | 0 | 0 | 0 | 0 | 0 | 0 | 0 | 0 |
| 6 | 9 | 0 | 11 | 0 | 0 | 0 | 0 | 0 | 0 | 0 |
| 7 | 0 | 0 | 0 | 0 | 0 | 0 | 0 | 0 | 0 | 0 |
| 8 | 26 | 0 | 0 | 0 | 0 | 0 | 0 | 0 | 0 | 0 |
| 9 | 0 | 19 | 0 | 0 | 5 | 57 | 0 | 0 | 21 | 91 |
| 10 | 0 | 0 | 0 | 0 | 0 | 0 | 0 | 0 | 0 | 0 |
| 11 | 7 | 0 | 0 | 0 | 0 | 0 | 0 | 0 | 0 | 0 |
| No. of positive | 7 | 2 | 1 | 0 | 1 | 1 | 0 | 0 | 1 | 1 |

| | | | | | | | | | | |
|---|---|---|---|---|---|---|---|---|---|---|
| * Mice were inoculated twice, because the dose of bacteria was too low at first time inoculation. | | | | | | | | | | |

### EXAMPLE 5

### C5a peptidase from group B streptococci is nearly identical in sequence to those from M12 and M49 group A streptococci.

The group B streptococci C5a peptidase (SCPB) gene was cloned, sequenced and compared to that from serotype group A streptococci M12 and M49. The entire *scpB* gene was amplified by PCR using primers which correspond to portions of the *scpA12* sequence using the method described in Example 4 above. The SCPB gene encodes an open reading frame (ORF) of 3450 bp which specifies a protein of 1150 amino acids with Mr of 126,237da. The amino acid sequence of SCPB is shown in Figure 2. Comparison of the *scpB* nucleotide and deduced amino acid sequence to those from M12 and M49 group A streptococci showed high similarities, 98% and 97%, respectively. *scpB* contained a 50 bp deletion which overlapped two of the C-terminal repeats, and had several other minor differences relative to *scpA* genes. Alignment of the sequences showed that *scpA12* is actually phylogenetically closer to *scpB* than it is to *scpA49*. Thirty strains, representing serotypes III, III/R, II, Ia/c, NT/c, NT/c/R1 carry a copy of *scpB*.

Recombinant SCP was expressed in *E*. *coli* using expression vector plasmid pGEX-4T-1 (ATCC accession number 98225) and was shown to be identical to the enzyme extracted from the parental group B streptococcal strain 78-471 (Type II a+ b). Western blot analysis suggested the recombinant SCP is identical to the C5ase enzyme previously purified from group B streptococci.

### EXAMPLE 6

### Intranasal Immunization with SCP Induces Serotype-Independent Immunity to Streptococcal Infections

**a) Bacterial strains.** Streptococcal strains CS101, CS210, and CS463 are spontaneous streptomycin resistant derivatives of serum opacity positive (OF⁺), class II, serotype M49, M2, and M11 strains, respectively. MJ3-15, described in Example 1 above, is strain CS101 with an internal inframe deletion in the SCPA49 gene. Streptococcal strains 90-131 and UAB200 are spontaneous streptomycin resistant derivatives of OF⁻, class I, serotype M1 and M6 human isolates of group A streptococci, respectively. Streptococci were cultured in Todd-Hewitt broth supplemented with 2% neopeptone or 1% yeast extract (THY) or on sheep blood agar. In some experiments streptococci were grown in the culture medium containing streptomycin (200 µg/ml) or erythromycin (1 µg/ml). *Escherichia coli* ER1821 (New England Biolabs, Inc., Beverly, Mass.) was used as the recipient for the thermosensitive suicide vector, plasmid pG⁺host5. pG⁺host5 was obtained from Appligene, Inc., Pleasanton, Calif. *E*. *coli* ER1821 containing plasmid pG⁺host5 was grown in Luria-Bertani broth containing erythromycin (Erm, 300µg/ml) at 39°C.
**b) Construction of the *scpA* insertion mutants.** The *scpA6* insertion mutant AK1.4 was constructed as described in Example 1 above. Recombinant plasmid DNA, pG::scpA1.2, contains an internal *Bgl*II*-Hind*III fragment *of scpA* gene. This plasmid was electroporated into UAB200 recipient cells and transformants were selected on THY agar plates containing erythromycin at 30°C. A chromosomal integrant of pG::scpA1.2, strain AK1.4, which resulted from recombination between the plasmid insert and the chromosomal *scpA6* was selected by growth on agar medium containing erythromycin at 39°C. Insertion into *scpA6* was confirmed by Southern blotting using *scpA* as the probe, and PCR using an M13 universal primer (5'-GTAAAACGACGGCCAGT-3') (SEQ. ID. No. 6), specific for the plasmid, and an *scpA* For835 primer (5'-AAGGACGACACATTGCGTA-3') (SEQ. ID. No. 7), specific for the chromosomal *scpA* of GAS.
**c) Construction, expression, and purification of ΔSCPA.** A 2.9 kb fragment of *scpA49* (from 1033 bp to 3941 bp) was amplified by PCR using an *scpA* forward primer containing a *Bam*HI recognition sequence (5'-CCCCCCGGATCCACCAAAACCCCACAAACTC-3') (SEQ. ID. No. 8) and an *scpA* reverse primer (5'-GAGTGGCCCTCCAATAGC-3') (SEQ. ID. No. 9). Sequences which code for the signal peptide and membrane anchor regions of the SCPA protein were deleted from the resulting PCR product. PCR products were digested with *Bam*HI and ligated to the thrombin recognition site of the glutathione S-transferase gene on the pGEX-4T-1 high expression vector from Pharmacia Inc.(Piscataway, NJ). The recombinant plasmid was transformed into *E*.*coli* DH5α. The ΔSCPA fusion protein from one transformant, *E.coli* (pJC6), was purified by affinity chromatography on a glutathione Sepharose 4B column. Following digestion with thrombin, thrombin was removed by chromatography on a benzamidine-Sepharose 6B column. Methods of expression and purification are described by the manufacturer. This affinity purified, truncated ΔSCPA protein lacked peptidase activity when tested by the PMN adherence assay (described in Example 1 above).
**d) Western blot techniques.** Mutanolysin extracts from streptococci were prepared as described in Example 1 above. Briefly, 100 ml of a streptococcal overnight culture was pelleted and washed twice in ice-cold 0.2M Na acetate (pH 5.2). The pellet was suspended in 1 ml of TE-sucrose buffer (1mM Tris, 1mM EDTA, and 20% sucrose) with 40µl of mutanolysin. After rotation at 37°C for 2 h, the mixture was centrifuged for 5 min. at 1500 × g. Phenylmethylsulfonyl fluoride (100mM) was added to the resulting supernatant. Western blots were performed as previously described in Example 1 above. Anti-SCPA antibody was prepared by immunization of rabbits with affinity purified recombinant ASCPA protein.
**e) PMN adherence and neutralization assays.** SCPA activity was measured by using a PMN adherence assay. Recombinant human C5a (rhC5a; C5788; Sigma, St. Louis, Mo) was incubated at 37°C for 45 min. with whole bacterial cells. Residual rhC5a was measured by its ability to activate PMNs, which become adherent to bovine serum albumin (BSA) coated wells of a microtiter plate. S.A. Booth et al., "Dapsone Suppresses Integrin-Mediated Neutrophil Adherence Function," J. Invest. Dermatol., 98, pp. 135-140 (1992). PMNs were isolated from fresh human blood by density gradient centrifugation in Ficoll Hypaque as described in Example 1 above. Neutralization of SCPA activity by rabbit anti-SCPA serum was assayed using the PMN adherence assay. Approximately 1 × 10⁷ heat-killed bacteria in 0.5% BSA-PBS were rotated with 1.4ml of rabbit anti-ΔSCPA49 serum or normal rabbit serum for 1 h. at 37°C. The bacteria were then resuspended in 40 µl of 0.5% BSA-PBS buffer and incubated with rhC5a for 45 min. before residual chemotaxin was measured by the PMN adherence assay.
**f) Phagocytosis assay.** *In vitro* human blood phagocytosis assays were performed as described in R.C. Lancefield, "Differentiation of Group A Streptococci with a Common R Antigen into Three Serological Types, with Special Reference to Bactericidal Test," J. Exp. Med., 106, pp. 525-685 (1957). Briefly, log-phase cultures of the group A streptococci were diluted in THY to 10³ to 10⁴ CFU/ml. One-tenth ml of diluted cultures and 0.9 ml of human blood were mixed and rotated at 37°C for 3 h. Initial viable counts and counts after 3 h. rotation were determined by plating diluted samples on blood agar.
**g) Mouse intranasal infection model.** Sixteen hour cultures of challenge streptococcal strains (1 × 10⁸ - 9 × 10⁸ CFU), grown in Todd-Hewitt broth containing 20% normal rabbit serum and resuspended in 10µl of PBS, were administered intranasally to 25g female CD1 (Charles River Breeding Laboratories, Inc., Wilmington, MA.) or BALB/c mice (Sasco, Omaha NE). Viable counts were determined by plating dilutions of cultures on blood agar plates. Throat swabs were taken daily from anesthetized mice for 6 to 10 days after inoculation and streaked onto blood agar plates containing 200ug/ml streptomycin. After overnight incubation at 37°C, the number of β-hemolytic colonies on plates were counted. All challenge strains were marked by streptomycin resistance to distinguish them from β-hemolytic bacteria which may be persist in the normal flora. Throat swabs were cultured on blood agar containing streptomycin. The presence of one β-hemolytic colony was taken as a positive culture.
**h) Immunization and challenge protocol.** Four week old, outbred, CD1 female mice were immunized by administration of 20µg of affinity purified ΔSCPA49 in 10µl PBS into each nostril. Mice were immunized 3 times on alternating days and boosted again three weeks after the third immunization. After two weeks rest, mice were again boosted. D. Bessen et al., "Influence of Intranasal Immunization with Synthetic Peptides Corresponding to Conserved Epitopes of M Protein on Mucosal Colonization by Group A Streptococci," Infect. Immun., 56, pp. 2666-2672 (1988). Control mice received only PBS. Prior to infection, all mice which were immunized with ΔSCPA protein were determined by ELISA to have high titers of antibodies against ΔSCPA antigen in their serum and saliva. Group A streptococci, strain CS101 (2.0 × 10⁸ CFU), CS210 (3.6 × 10⁸ CFU), CS463 (7.8 × 10⁸ CFU), 90-131 (3.4 × 10⁸ CFU), and UAB200 (9.6 × 10⁸ CFU) were used to intranasally challenge the mice 7 days after the last vaccine booster. Animal studies were performed according to National Institutes of Health guidelines.
**i) Sample collection and ELISA.** Blood and saliva samples were collected from anesthetized mice after immunization. All sera were tested for the presence of SCPA49 antibodies by ELISA, as previously described. S.P. O'Connor et al., "The Human Antibody Response to Streptococcal C5a Peptidase," J. Infect. Dis., 163, pp. 109-116 (1990). Purified SCPA49 protein was bound to microtiter wells by addition of 500ng of purified protein in 0.05M bicarbonate buffer (pH 9.6). After overnight incubation at 4°C the wells were washed, then blocked with 0.5% BSA in PBS for 1 hour. Salivation was stimulated in mice by injection of 100 µl of a 0.1% pilocarpine (Sigma) solution subcutaneously. Saliva samples were collected and spun at 14,000 rpm for 5 min in an Eppendorf microcentrifuge. The supernatants were tested for the presence of secretory IgA against ASCPA49 protein by ELISA. ELISA titers represent the highest dilution of individual serum and saliva which had an OD₄₀₅ ≥ 0.1.
**j) Statistical analyses.** The χ² test was used to analyze the data from animals experiments. A *P* value of < 0.05 was considered significant.

First, the ability of wild type and isogenic SCPA⁻ mutant streptococci to colonize the nasopharynx of mice was studied. Mutanolysin extracts of cell surface proteins from parent and mutant cultures were analyzed by Western blot using SCPA specific serum. Mutants were confirmed to lack SCPA. Extracts of SCPA⁻ mutants AK1.4 and MJ3-15 did not react with anti-SCPA serum. SCPA proteins of the expected size were observed in extracts from the wild type strains CS101 and UAB200. Failure of mutant strains AK1.4 and MJ3-15 to produce C5a peptidase activity was verified by comparing their capacity to destroy rhC5a. Exposure of isolated PMNs to rhC5a induced them to become adherent to BSA coated microtiter wells. Incubation with streptococci or purified SCPA specifically cleaved rhC5a and altered its potential to activate PMNs. PMNs that responded to residual rhC5a and bound to BSA coated wells, were stained, then measured spectrophotometrically. Incubation of rhC5a with parent cultures, UAB200 and CS101, destroyed rhC5a, which inhibited PMN adherence by 58.8% and 54.5%, respectively. In contrast SCPA⁻ mutants, AK1.4 and MJ3-15, did not alter rhC5a or adherence of PMNs to BSA coated wells (Table 5). This experiment confirmed the above Western blots and demonstrated that SCPA⁻ cultures lack other proteases which might degrade rhC5a.

**Table 5.**

| Phagocytosis assay and PMN adherence assay of wild type and mutant strains | | | | | |
|---|---|---|---|---|---|
| Strain | Description | Colony forming units (cfu)/ml | | Fold increase in cfu/ml | Percent inhibition of C5a induced PMN adherence* |
| | | Time=0h | Time=3h | | |
| UAB200 | M6⁺, SCPA⁺ | 1.8 x 10³ | 7.2 x 10⁴ | 40 | 58.8 |
| AK1.4 | M6⁺, SCPA⁻ | 1.2 x 10³ | 4.5 x 10⁴ | 37.5 | 0 |
| | | | | | |
| CS101 | M49⁺, SCPA⁺ | 1.0 x 10⁴ | 4.9 x 10⁵ | 49 | 54.5 |
| MJ3-15 | M49⁺, SCPA⁻ | 1.5 x 10⁴ | 2.1 x 10⁵ | 14 | 0 |

| | | | | | |
|---|---|---|---|---|---|
| *Percent inhibition = [(OD₅₇₀ₙₘ of PMNs activated by C5a alone - OD₅₇₀ₙₘ PMNs activated by C5a preincubated with bacteria / OD₅₇₀ₙₘ of PMNs activated by C5a alone)] x 100%. | | | | | |

Although M protein expression was not expected to be influenced by mutations in *scpA*, assays were performed to assess whether SCPA⁻ mutant streptococci still expressed M protein and had the ability to resist phagocytosis. Growth of streptococci in fresh human blood during 3 hours incubation is indicative of antiphagocytic M protein on their surface. R.C. Lancefield, "Differentiation of Group A Streptococci with a Common R Antigen into Three Serological Types, with Special Reference to Bactericidal Test," J. Exp. Med., 106, pp. 525-685 (1957). As expected, parent streptococci, UAB200 and CS101, increased 40 and 49 fold, respectively (Table 5). The M⁺ SCPA⁻ cultures, strains AK1.4 and MJ3-15, increased 37.5 and 14-fold, respectively, confirming that *scpA* mutations had little effect on M protein expression or resistance to phagocytosis in whole human blood. The somewhat poorer growth of both mutant strains in rotated blood was reproducible and unexpected. The growth rates of mutant and parent cultures in human plasma were indistinguishable. It is possible that inactivation of SCPA allowed C5a to accumulate in rotated blood which in turn activated PMNs. Activated PMNs are more phagocytic and better able to kill M⁺ streptococci. Surface protein extracts contain M6 and M49 antigen when analyzed by Western blot using anti-M49 and anti-M6 antisera, confirming that mutations in SCPA did not alter M protein expression.

Next, it was determined if the C5a peptidase is required for nasopharyngeal colonization. CD-1 outbred mice were inoculated intranasally to evaluate the relative capacity of wild type and SCPA⁻ streptococci to colonize the nasopharynx. Throat swabs were taken daily for 1 to 10 days from anesthetized mice and streaked onto blood agar plates containing antibiotics selective for the strain in question. BALB/c mice were used for the M6 strain UAB200 infections in order to conform with earlier studies using this strain. D. Bessen et al., "Influence of Intranasal Immunization with Synthetic Peptides Corresponding to Conserved Epitopes of M Protein on Mucosal Colonization by Group A Streptococci," Infect. Immun., 56, pp. 2666-2672 (1988). The inoculum size which resulted in colonization of approximately 50% of the mice for up to five days was first determined. Significant differences between M⁺ SCPA⁺ and M⁺ SCPA⁻ type 49 streptococci were observed on days 3 to 9 after inoculation. By day four, 50% (9 of 18) of mice infected with strain CS101 still produced positive throat cultures; whereas, only 11% (2 of 18) of mice infected with MJ3-15 retained streptococci in their throats. Differences in the number of colonies on the blood agar plates were also consistent with more rapid clearance of M⁺ SCPA⁻ streptococci. 59% (31 of 54) of positive cultures from mice infected with wild type streptococci contained more than 100 CFU; whereas only 14% (2 of 14) of positive cultures from animals infected with SCPA⁻ streptococci, contained more than 100 CFU (statistically significant to P<0.001). Moreover, 22% (4 of 18) of the mice died from infection with M⁺ SCPA⁺ streptococci. None of the mice died from infection with M⁺ SCPA⁻ streptococci. This difference is also statistically significant (P<0.05). Comparison of SCPA49⁺ and SCPA49⁻ variants of strain CS101 was repeated two more times with similar results.

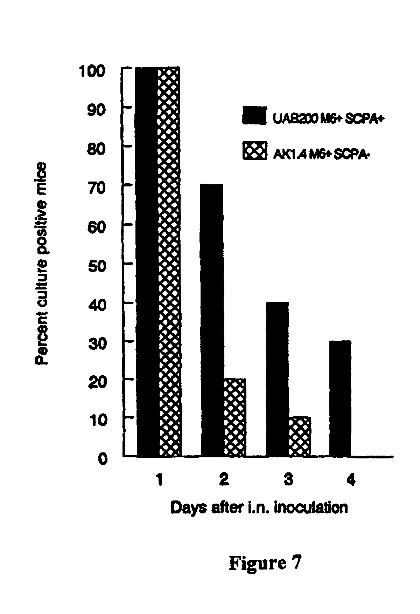

Because the spectrum of disease caused by OF⁺ and OF⁻ strains differ significantly, the impact of SCPA on colonization by an OF⁻ serotype, type M6 was also investigated (Fig. 7). Again the SCPA6⁻ strain AK1.4 was cleared more rapidly than the parent strain UAB200. Four days after infection all mice had completely cleared SCPA⁻ streptococci from their throat; whereas, 30% of mice infected with wild type streptococci remained culture positive. Greater than 98% of all positive cultures had more than one colony on the blood agar plate. In this experiment all mice were free of streptococci by the 5th day post inoculation.

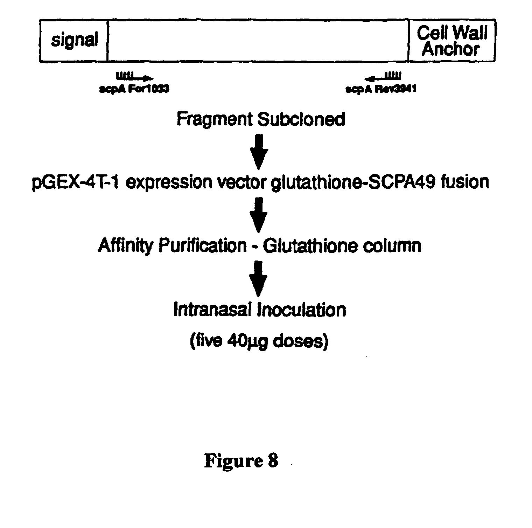

The next step was to determine if SCP could be used to immunize an animal. First, an enzymatically inactive form of SCPA49 protein was constructed for use in immunization studies. A 2908 bp *scpA49* fragment with additional *Bam*HI recognition sequences was obtained by PCR and ligated into plasmid pGEX-4T-1, which had been digested with *Bam*HI and *Sma*I. In this construct, plasmid pJC6, the *scpA49* sequence was fused in-frame to the glutathione transferase gene. The streptococcal insert did not include the *scpA* signal or cell wall anchor sequences (Fig. 8). Vaccine preparations of purified ΔSCPA49 protein were evaluated by SDS-PAGE and Western blot to confirm purity. Several protein bands in the purified ΔSCPA49 preparation reacted with polyclonal rabbit antiserum directed against recombinant ΔSCPA49 protein. The size of the major band was approximately 100KD, the estimated size of the deletion form of SCPA49. Smaller bands were assumed to be degradation products of SCPA, a common feature of over expressed proteins in *E*.*coli*. The antiserum did not reacted with any protein isolated from *E.coli* DH5α (pGEX-4T-1) without a streptococcal insert. The procedure described above routinely yielded 2-3mg of highly pure ΔSCPA49 protein from one liter of culture. The purified ΔSCPA49 protein was found to lack C5a peptidase activity when assayed by the PMN adherence assay.

Second, the immunogenicity of the subunit ΔSCPA49 vaccine was evaluated. Rabbits were immunized with purified ΔSCPA49. The rabbits developed high levels of antibodies against ΔSCPA49 protein as determined by ELISA. Although the purified ΔSCPA49 immunogen lacked functional activity, hyperimmune rabbit antiserum could neutralize the peptidase activity of purified wild type SCPA49 enzyme *in vitro*. Moreover, undiluted rabbit antiserum against ΔSCPA49 protein was able to neutralize C5a peptidase activity associated with heterologous serotypes. C5a peptidase activity associated with intact M1, M6 and M12 streptococci was inhibited by this antiserum, confirming that antibody against ASCPA49 protein has broad cross-reactivity against a number of different serotypes.

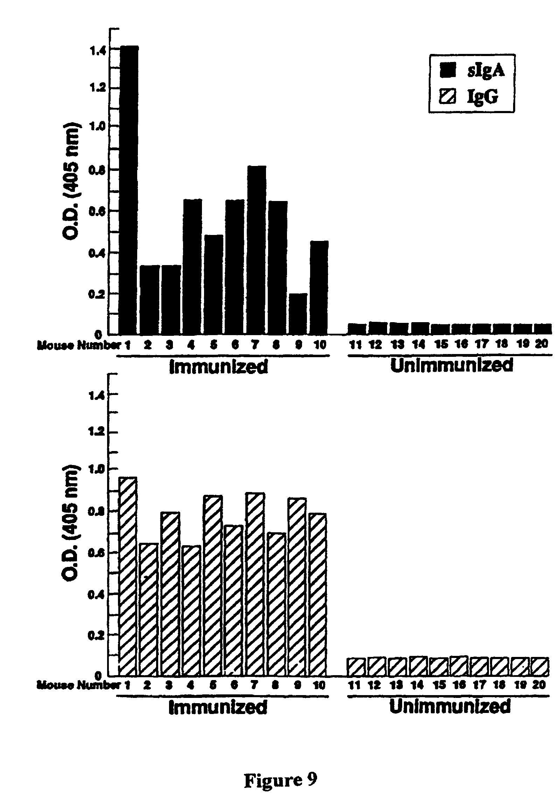

Also, serum and saliva samples were obtained from ten immunized and ten control mice to assess the immunogenicity of ΔSCPA49 protein when administered via the intranasal route without adjuvants. Mice which were immunized with purified ΔSCPA49 protein developed high titers of SCPA-specific IgG in their sera, compared to control mice immunized with PBS. Titers of serum IgG directed against ΔSCPA49 ranged from 1:10,240 to 1:20,480. In contrast, SCPA49-specific IgG titer of control mice was not detectable in sera. Mice immunized with purified ΔSCPA49 protein also showed a significant increase in SCPA49-specific salivary IgA relative to control mice. Specific IgA titers in saliva of immunized mice were greater than 1:16. In contrast, IgA directed against SCPA49 in the saliva of control mice was not detectable. The relative concentration of IgG and IgA in serum diluted 1/2560 and saliva diluted 1/2, respectively, are shown in Figure 9. These results demonstrate that purified ΔSCPA49 protein is an effective immunogen for the induction of specific systemic and secretory antibody responses in mice when administered intranasally.

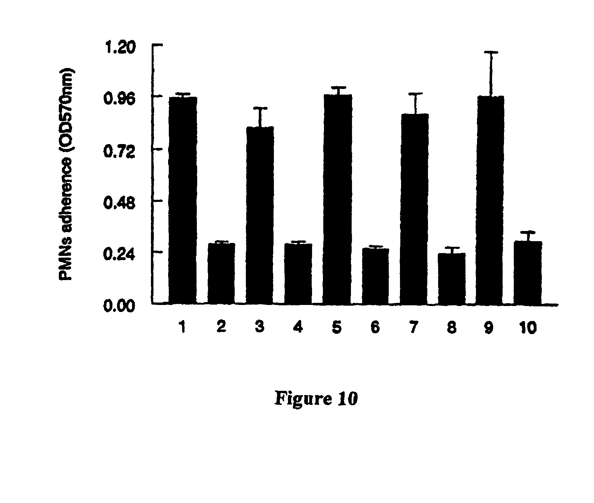

Third, experiments were performed to determine whether immunization with the C5a peptidase would enhance clearance of streptococci from the nasopharynx. Both hyperimmune rabbit and human sera that contain high levels of anti-SCPA antibody can neutralize SCPA activity *in vitro.* S.P. O'Connor et al., "The Human Antibody Response to Streptococcal C5a Peptidase," J. Infect. Dis., 163, pp. 109-116 (1990). The fact that SCPA significantly facilitates colonization of the oral mucosa suggests that immunization of mice with purified ΔSCPA49 could reduce the capacity of streptococci to colonize the nasopharynx. Mice were immunized intranasally with affinity purified, genetically inactivated SCPA to test this possibility. The truncated protein, ΔSCPA49, was administered intranasally without adjuvants or carriers. Pharyngeal colonization of vaccinated mice by wild type M⁺ SCPA⁺ streptococci differed significantly from those immunized with PBS in three independent experiments using mice vaccinated with two different preparations of purified ΔSCPA49 protein (Representative data are shown in Figure 10). Only one of 13 mice immunized with ΔSCPA49 protein was culture positive for streptococci ten days after inoculation (Fig. 10). In contrast, 30-58% of the non-vaccinated controls remained culture positive for six days, and some were still positive ten days after infection. The numbers of β-hemolytic, streptomycin resistant colonies on blood agar plates also showed a significant difference between ΔSCPA49 vaccinated and control mice. Different sets of immunized mice cleared serotype M49 streptococci significantly more rapidly from their nasopharynx than non-immunized control.

Last, it was examined whether SCP of one serotype would vaccinate animals against infection from other serotypes. There are more than 80 different serotypes of group A streptococci. An effective vaccine should prevent infection by more than one streptococcal serotype. Cross-protection was observed against serotypes M2, M11, M1, and M6 group A streptococcal colonization. The fact that rabbit serum directed against ΔSCPA49 protein from serotype M49 streptococci neutralized peptidase activity associated with several different serotypes suggested that intranasal immunization with a single subunit vaccine might reduce or eliminate pharyngeal colonization by those serotypes. To explore this possibility four groups of twenty mice were immunized by intranasal inoculation with affinity purified ΔSCPA49 protein as described above. Control mice received PBS. Prior to being challenged with streptococci, serum and saliva samples from randomly chosen, immunized and control mice were assayed for anti-SCPA antibody. All immunized mice tested had developed a strong serum and measurable salivary antibody response. Pharyngeal colonization of mice immunized with ΔSCPA49 protein by strains of all four serotypes was reduced relative to non-immunized controls. Differences were most significant on days 3 and 5 after inoculation (Table 6).

**Table 6.**

| Immune protectivity is serotype independent | | | | | | | | |
|---|---|---|---|---|---|---|---|---|
| Day 3 after inoculation | | | | | Day 5 after inoculation | | | |
| | Nonimmune | | Immune | | Nonimmune | | Immune | |
| | (+/total) | % | (+/total) | % | (+/total) | % | (+/total) | % |
| M2 | 10/19 | 52.6 | 2/19* | 10.5 | 3/19 | 15.8 | 1/19 | 5.2 |
| M11 | 17/20 | 85 | 11/20* | 55 | 8/20 | 40 | 2/20* | 10 |
| M1 | 16/19 | 84.2 | 11/19 | 57.9 | 7/19 | 37 | 2/19* | 10.5 |
| M6 | 14/20 | 70 | 12/19 | 63.2 | 8/20 | 40 | 4/19 | 21.1 |

| | | | | | | | | |
|---|---|---|---|---|---|---|---|---|
| + means culture positive mice. * Differences between immunized and non-immunized mice are statistically significant (P<0.05). P values were calculated by x² analysis. | | | | | | | | |

Statistically significant differences were observed between immunized and control mice inoculated with serotype M2, M11 and M 1 strains. However, the OF⁺ serotypes M2 and M11 were more efficiently eliminated by immunized mice than were the OF⁻ strains, M I and M6. M 1 streptococcal colonization of immunized mice was significantly reduced relative to control mice. Only 10.5% of the immunized mice were culture positive by day 5 post-infection. In contrast, 37% of the control mice were culture positive with this strain. Although immunized mice appeared to clear M6 streptococci more rapidly, the differences were not statistically significant. As in previous experiments the number of β-hemolytic streptococcal colonies on blood agar plates were significantly fewer in samples taken from vaccinated mice than those taken from control animals. Thus, the ASCPA 49 protein was an effective vaccine that provided cross-protection against other streptococcal serotypes.

The invention has been described with reference to various specific and preferred embodiments and techniques. However, it should be understood that many variations and modifications may be made while remaining within the scope of the invention.

## Claims

1. A vaccine comprising an immunogenic amount of an enzymatically inactive streptococcal C5a peptidase,
or a fragment, variant or mutant thereof, which amount is effective to immunize a susceptible mammal against β-hemolytic *Streptococcus* in combination with a physiologically-acceptable, non-toxic vehicle.

2. The vaccine of claim 1, which further comprises an effective amount of an immunological adjuvant.

3. The vaccine of any one of claims 1 or 2, wherein said mammal is selected from the group consisting of human, dog, bovine, porcine and horse.

4. The vaccine of claim 3, wherein said mammal is human.

5. The vaccine of any one of claims 1 to 4, wherein said β-hemolytic *Streptococcus* is selected from the group consisting of group A *Streptococcus*, group B *Streptococcus*, group C *Streptococcus* and group G *Streptococcus*.

6. The vaccine of claim 5, wherein said β-hemolytic *Streptococcus* is group A *Streptococcus*.

7. The vaccine of any one of claims 1 to 6, which comprises a recombinant streptococcal C5a peptidase, or fragment, variant or mutant thereof, conjugated or linked to a peptide.

8. The vaccine of any one of claims 1 to 6, which comprises said streptococcal C5a peptidase, or fragment, variant or mutant thereof, conjugated or linked to a polysaccharide.

9. The vaccine of any one of claims 1 to 8, which is suitable for administration by subcutaneous or intramuscular injection.

10. The vaccine of any one of claims 1 to 8, which is suitable for administration by oral ingestion.

11. The vaccine of any one of claims 1 to 8, which is suitable for administration intranasally.

12. Use of an enzymatically inactive streptococcal C5a peptidase, or a fragment, variant or mutant thereof, in combination with a physiologically acceptable, non-toxic vehicle for the preparation of a vaccine for protecting a susceptible mammal against *Streptococcus* colonization or infection.

13. The use of claim 12 wherein said vaccine is as defined in any of claims 1 to 11.

14. An enzymatically inactive streptococcal C5a peptidase, or an immunogenically active fragment, variant or mutant thereof, for use in therapy.

## Patentansprüche

1. Vakzine umfassend eine immunogene Menge einer enzymatisch inaktiven *Streptococcus* C5a Peptidase, oder ein Fragment, eine Variante oder Mutante davon, wobei die Menge ausreicht, um einen anfälligen Säuger gegen β-hämolytischen *Streptococcus* zu immunisieren, in Kombination mit einem physiologisch verträglichen, nicht-toxischen Träger.

2. Vakzine nach Anspruch 1, welche außerdem eine wirksame Menge eines immunologischen Adjuvants umfasst.

3. Vakzine nach einem der Ansprüche 1 oder 2, wobei der Säuger ausgewählt ist unter Mensch, Hund, Rind, Schwein und Pferd.

4. Vakzine nach Anspruch 3, wobei der Säuger ein Mensch ist.

5. Vakzine nach einem der Ansprüche 1 bis 4, wobei der β-hämolytische *Streptococcus* ausgewählt ist unter *Streptococcus* der Gruppe A, *Streptococcus* der Gruppe B, *Streptococcus* der Gruppe C und *Streptococcus* der Gruppe G.

6. Vakzine nach Anspruch 5, wobei das β-hämolytische Streptococcus ein Streptococcus der Gruppe A ist.

7. Vakzine nach einem der Ansprüche 1 bis 6, welche eine rekombinante *Streptococcus* C5a Peptidase oder ein Fragment, eine Variante oder Mutante davon, konjugiert oder verknüpft mit einem Peptid umfasst.

8. Vakzine nach einem der Ansprüche 1 bis 6, welche eine *Streptococcus* C5a Peptidase oder ein Fragment, eine Variante oder Mutante davon, konjugiert oder verknüpft mit einem Polysaccharid umfasst.

9. Vakzine nach einem der Ansprüche 1 bis 8, welche zur Verabreichung durch subkutane oder intramuskuläre Injektion geeignet ist.

10. Vakzine nach einem der Ansprüche 1 bis 8, welche zur Verabreichung durch orale Aufnahme geeignet ist.

11. Vakzine nach einem der Ansprüche 1 bis 8, welche zur intranasalen Verabreichung geeignet ist.

12. Verwendung einer enzymatisch inaktiven *Streptococcus* C5a Peptidase oder eines Fragmentes, einer Variante oder Mutante davon, in Kombination mit einem physiologisch verträglichen, nicht-toxischen Träger, zur Herstellung einer Vakzine zum Schutz eines anfälliges Säugers gegen eine *Streptococcus*-Kolonisierung oder-Infektion.

13. Verwendung nach Anspruch 12, wobei die Vakzine nach einem der Ansprüche 1 bis 11 definiert ist.

14. Enzymatisch inaktive *Streptococcus* C5a Peptidase oder ein aktives Fragment, eine aktive Variante oder Mutante davon, zur Verwendung in der Therapie.

## Revendications

1. Vaccin comprenant une quantité immunogène d'une peptidase C5a streptococcique enzymatiquement inactive,
ou un fragment, une variante ou un mutant de celle-ci, dont la quantité est efficace pour immuniser contre *Streptococcus* β-hémolytique un mammifère sensible à celui-ci, en combinaison avec un véhicule non toxique physiologiquement acceptable.

2. Vaccin selon la revendication 1 qui comprend en outre une quantité efficace d'un adjuvant immunologique.

3. Vaccin selon l'une quelconque des revendications 1 ou 2, dans lequel ledit mammifère est choisi dans le groupe composé des êtres humains, des chiens, des bovins, des porcins et des chevaux.

4. Vaccin selon la revendication 3, dans lequel ledit mammifère est un être humain.

5. Vaccin selon l'une quelconque des revendications 1 à 4, dans lequel ledit *Streptococus* β-hémolytique est choisi dans le groupe composé de *Streptococcus* du groupe A, de *Streptococcus* du groupe B, de *Streptococcus* du groupe C et de *Streptococcus* du groupe G.

6. Vaccin selon la revendication 5, dans lequel ledit *Streptococcus β*-hémolytique est *Streptococcus* du groupe A.

7. Vaccin selon l'une quelconque des revendications 1 à 6, qui comprend une peptidase C5a streptococcique recombinante, ou un fragment, une variante ou un mutant de celle-ci, conjuguée ou liée à un peptide.

8. Vaccin selon l'une quelconque des revendications 1 à 6, qui comprend ladite peptidase C5a streptococcique, ou un fragment, une variante ou un mutant de celle-ci, conjuguée ou liée à un polysaccharide.

9. Vaccin selon l'une quelconque des revendications 1 à 8 convenant à une administration par injection sous-cutanée ou intramusculaire.

10. Vaccin selon l'une quelconque des revendications 1 à 8 convenant à une administration par ingestion orale.

11. Vaccin selon l'une quelconque des revendications 1 à 8 convenant à une administration intra-nasale.

12. Utilisation d'une peptidase C5a streptococcique enzymatiquement inactive, ou d'un fragment, d'une variante ou d'un mutant de celle-ci, en combinaison avec un véhicule non toxique physiologiquement acceptable pour la préparation d'un vaccin destiné à protéger contre une colonisation ou une infection à *Streptococcus* un mammifère sensible à ceux-ci.

13. Utilisation de la revendication 12, dans laquelle ledit vaccin est tel qu'il est défini dans l'une quelconque des revendications 1 à 11.

14. Peptidase C5a streptococcique enzymatiquement inactive, ou un fragment, une variante ou un mutant de celle-ci actif d'un point de vue immunogène, destinée à être utilisée en thérapie.


### SEQUENCE LISTING

(1) GENERAL INFORMATION:
   (i) APPLICANT: Regents of the University of Minnesota
   (ii) TITLE OF INVENTION: STREPTOCOCCAL C5a PEPTIDASE VACCINE
   (iii) NUMBER OF SEQUENCES: 9
   (iv) CORRESPONDENCE ADDRESS:
      (A) NAME: SCHWEGMAN, LUNDBERG, WOESSNER & KLUTH, P.A.
      (B) STREET: P.O. Box 2938
      (C) CITY: MINNEAPOLIS
      (D) STATE: MINNESOTA
      (E) COUNTRY: USA
      (F) POSTAL CODE (ZIP): 55402
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) CURRENT APPLICATION DATA:
      APPLICATION NUMBER: UNKNOWN
   (vii) PRIOR APPLICATION DATA:
      (A) APPLICATION NUMBER: 08/589,756
      (B) FILING DATE: January 22, 1996
   (viii) ATTORNEY/AGENT INFORMATION:
      (A) NAME; Ann S. Viksnins
      (B) REGISTRATION NUMBER: 37,748
      (C) REFERENCE/DOCKET NUMBER: 600.349WO1
   (ix) TELECOMMUNICATION INFORMATION:
      (A) TELEPHONE: 612-359-3260
      (B) TELEFAX: 612-359-3263
      (C) TELEX:
(2) INFORMATION FOR SEQ ID NO: 1:
   (i) SEQUENCE CHARACTERISTICS:
      (A) LENGTH: 1164 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 1:
   Leu Arg Lys Lys Gln Lys Leu Pro Phe Asp Lys Leu Ala Ile Ala Leu
   1 5 10 15
   Met Ser Thr Ser Ile Leu Leu Asn Ala Gln Ser Asp Ile Lys Ala Asn
   20 25 30
   Thr Val Thr Glu Asp Thr Pro Ala Thr Glu Gln Ala Val Glu Thr Pro
   35 40 45
   Gln Pro Thr Thr Val Ser Glu Glu Val Pro Ser Ser Lys Glu Thr Lys
   50 55 60
   Thr Pro Gln Thr Pro Asp Asp Ala Glu Glu Thr Val Ala Asp Asp Ala
   65 70 75 80
   Asn Asp Leu Ala Pro Gln Ala Pro Ala Lys Thr Pro Asp Thr Ser Ala
   85 90 95
   Thr Ser Lys Ala Thr Ile Arg Asp Leu Asn Asp Pro Ser Gln Val Lys
   100 105 110
   Thr Leu Gln Glu Lys Ala Gly Lys Gly Ala Gly Thr Val Val Ala Val
   115 120 125
   Ile Asp Ala Gly Phe Asp Lys Asn His Glu Ala Trp Arg Leu Thr Asp
   130 135 140
   Lys Ala Lys Ala Arg Tyr Gln Ser Lys Glu Asp Leu Glu Lys Ala Lys
   145 150 155 160
   Lys Glu His Gly Ile Thr Tyr Gly Glu Trp Val Asn Asp Lys Val Ala
   165 170 175
   Tyr Tyr His Asp Tyr Ser Lys Asp Gly Lys Thr Ala Val Asp Gln Glu
   180 185 190
   His Gly Thr His Val Ser Gly Ile Leu Ser Gly Asn Ala Pro Ser Glu
   195 200 205
   Thr Lys Glu Pro Tyr Arg Leu Glu Gly Ala Met Pro Glu Ala Gln Leu
   210 215 220
   Leu Leu Met Arg Val Glu Ile Val Asn Gly Leu Ala Asp Tyr Ala Arg
   225 230 235 240
   Asn Tyr Ala Gln Ala Ile Arg Asp Ala Val Asn Leu Gly Ala Lys Val
   245 250 255
   Ile Asn Met Ser Phe Gly Asn Ala Ala Leu Ala Tyr Ala Asn Leu Pro
   260 265 270
   Asp Glu Thr Lys Lys Pro Phe Val Tyr Ala Lys Ser Lys Gly Val Arg
   275 280 285
   Ile Val Thr Thr Ala Gly Asn Asp Ser Ser Phe Gly Gly Lys Thr Arg
   290 295 300
   Leu Pro Leu Ala Asp His Pro Asp Tyr Gly Val Val Gly Thr Pro Ala
   305 310 315 320
   Ala Ala Asp Ser Thr Leu Thr Val Ala Ser Tyr Ser Pro Asp Asn Gln
   325 330 335
   Leu Thr Glu Thr Ala Met Val Lys Thr Asp Asp Gln Gln Asp Lys Glu
   340 345 350
   Met Pro Val Leu Ser Thr Asn Arg Phe Glu Pro Asn Lys Ala Tyr Asp
   355 360 365
   Tyr Ala Tyr Ala Asn Arg Gly Met Lys Glu Asp Asp Phe Lys Asp Val
   370 375 380
   Lys Gly Lys Ile Ala Leu Ile Glu Arg Ser Asp Ile Asp Phe Thr Asp
   385 390 395 400
   Lys Ile Ala Asn Ala Lys Lys Ala Gly Ala Val Gly Val Leu Ile Tyr
   405 410 415
   Asp Asn Gln Asp Lys Gly Phe Pro Ile Glu Leu Pro Asn Val Asp Gln
   420 425 430
   Met Pro Ala Ala Phe Ile Ser Arg Lys Asp Gly Leu Leu Leu Lys Asp
   435 440 445
   Asn Ser Gln Lys Thr Ile Thr Phe Asn Ala Thr Pro Lys Val Leu Pro
   450 455 460
   Thr Ala Ser Gly Thr Lys Leu Ser Arg Phe Ser Ser Trp Gly Leu Thr
   465 470 475 480
   Ala Asp Gly Asn Ile Lys Pro Asp Ile Ala Ala Pro Gly Gln Asp Ile
   485 490 495
   Leu Ser Ser Ala Ala Asn Asn Lys Tyr Ala Lys Leu Ser Gly Thr Ser
   500 505 510
   Met Ser Ala Pro Leu Val Ala Val Ile Met Gly Leu Leu Gln Lys Gln
   515 520 525
   Tyr Glu Thr Gln Tyr Pro Asp Met Thr Gln Ser Glu Arg Leu Asp Leu
   530 535 540
   Ala Lys Lys Val Leu Met Ser Ser Ala Thr Ala Leu Tyr Asp Glu Asp
   545 550 555 560
   Glu Lys Ala Tyr Phe Ser Pro Arg Gln Gln Gly Ala Gly Ala Val Asp
   565 570 575
   Ala Lys Lys Ala Ser Glu Ala Thr Met Tyr Val Thr Asp Lys Asp Asn
   580 585 590
   Thr Ser Ser Lys Val His Leu Asn Asn Val Ser Asp Lys Phe Glu Val
   595 600 605
   Thr Val Thr Val His Asn Lys Ser Asp Lys Pro His Glu Leu Tyr Tyr
   610 615 620
   Gln Ala Thr Val Gln Thr Asp Lys Val Asp Gly Lys His Phe Ala Leu
   625 630 635 640
   Ala Pro Lys Ala Leu Ile Glu Thr Ser Trp Gln Lys Ile Thr Ile Pro
   645 650 655
   Ala Asn Ser Ser Lys Gln Val Thr Ile Pro Ile Asp Ile Ser Gln Phe
   660 665 670
   Ser Lys Asp Leu Leu Ala Gln Met Lys Asn Gly Tyr Phe Leu Glu Gly
   675 680 685
   Phe Val Arg Ile Lys Gln Asp Pro Thr Lys Glu Glu Leu Met Ser Ile
   690 695 700
   Pro Tyr Ile Gly Phe Arg Gly Asp Phe Gly Asn Leu Ser Ala Leu Glu
   705 710 715 720
   Lys Pro Leu Tyr Asp Ser Lys Asp Gly Ser Ser Tyr Tyr His Glu Glu
   725 730 735
   Ile Ser Asp Ala Lys Asp Gln Leu Asp Gly Asp Gly Leu Gln Phe Tyr
   740 745 750
   Ala Leu Lys Asn Asp Phe Thr Ala Leu Thr Thr Glu Ser Asn Pro Trp
   755 760 765
   Thr Ile Ile Asn Val Val Lys Glu Gly Val Glu Asn Ile Glu Asp Ile
   770 775 780
   Glu Ser Ser Glu Ile Thr Glu Thr Ile Phe Ala Gly Thr Phe Ala Lys
   785 790 795 800
   Gln Asp Asp Asp Arg His Tyr Tyr Ile His Arg His Ala Asn Gly Lys
   805 810 815
   Pro Tyr Ala Ala Ile Ser Pro Asn Gly Asp Gly Asn Arg Asp Tyr Val
   820 825 830
   Gln Phe His Gly Thr Phe Leu Arg Asn Ala Lys Asn Leu Val Ala Glu
   835 840 845
   Val Leu Asp Lys Glu Gly Asn Val Val Trp Thr Ser Glu Val Thr Glu
   850 855 860
   Gln Val Val Lys Asn Tyr Asn Asn Asp Leu Ala Ser Thr Leu Gly Ser
   865 870 875 880
   Thr Arg Phe Glu Ile Ser Arg Trp Asp Gly Lys Asp Lys Asp Ala Lys
   885 890 895
   Val Val Ala Asn Gly Thr Tyr Thr Tyr Arg Val Arg Tyr Thr Pro Ile
   900 905 910
   Ser Ser Gly Ala Lys Glu Gln His Thr Asp Phe Asp Val Ile Val Asp
   915 920 925
   Asn Thr Thr Pro Glu Val Ala Thr Ser Ala Thr Phe Ser Thr Glu Asp
   930 935 940
   Arg Arg Leu Thr Leu Ala Ser Lys Pro Gln Thr Ser Gln Pro Val Tyr
   945 950 955 960
   Arg Glu Arg Ile Ala Tyr Thr Tyr Met Asp Glu Asp Leu Pro Thr Thr
   965   970 975
   Glu Tyr Ile Ser Pro Asn Glu Asp Gly Thr Phe Thr Leu Pro Glu Glu
   980 985 990
   Ala Glu Thr Met Glu Gly Ala Thr Val Pro Leu Lys Met Ser Asp Phe
   995 1000 1005
   Thr Tyr Val Val Glu Asp Met Ala Gly Asn Ile Thr Tyr Thr Pro Val
   1010 1015 1020
   Thr Lys Leu Leu Glu Gly His Ser Asn Lys Pro Glu Gln Asp Gly Ser
   1025 1030 1035 1040
   Asp Gln Ala Pro Asp Lys Lys Pro Glu Thr Lys Pro Glu Gln Asp Gly
   1045 1050 1055
   Ser Asp Gln Ala Pro Asp Lys Lys Pro Glu Thr Lys Pro Gly Gln Asp
   1060 1065 1070
   Gly Ser Gly Gln Thr Pro Asp Lys Lys Pro Glu Thr Lys Pro Glu Lys
   1075 1080 1085
   Asp Ser Ser Gly Gln Thr Pro Gly Lys Thr Pro Gln Lys Gly Gln Pro
   1090 1095 1100
   Ser Arg Thr Leu Glu Lys Arg Ser Ser Lys Arg Ala Leu Ala Thr Lys
   1105 1110 1115 1120
   Ala Ser Thr Arg Asp Gln Leu Pro Thr Thr Asn Asp Lys Asp Thr Asn
   1125 1130 1135
   Arg Leu His Leu Leu Lys Leu Val Met Thr Thr Phe Phe Leu Gly Leu
   1140 1145 1150
   Val Ala His Ile Phe Lys Thr Lys Arg Thr Glu Asp
   1155 1160
(2) INFORMATION FOR SEQ ID NO: 2:
   (i) SEQUENCE CHARACTERISTICS:
      (A) LENGTH: 1167 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 2:
   Leu Arg Lys Lys Gln Lys Leu Pro Phe Asp Lys Leu Ala Ile Ala Leu
   1               5                   10                  15
   Met Ser Thr Ser Ile Leu Leu Asn Ala Gln Ser Asp Ile Lys Ala Asn
               20                  25                  30
   Thr Val Thr Glu Asp Thr Pro Val Thr Glu Gln Ala Val Glu Thr Pro
           35                  40                  45
   Gln Pro Thr Ala Val Ser Glu Glu Val Pro Ser Ser Lys Glu Thr Lys
       50                  55                  60
   Thr Pro Gln Thr Pro Asp Asp Ala Glu Glu Thr Ile Ala Asp Asp Ala
   65                  70                  75                  80
   Asn Asp Leu Ala Pro Gln Ala Pro Ala Lys Thr Ala Asp Thr Pro Ala
                   85                  90                  95
   Thr Ser Lys Ala Thr Ile Arg Asp Leu Asn Asp Pro Ser Gln Val Lys
               100                 105                 110
   Thr Leu Gln Glu Lys Ala Gly Lys Gly Ala Gly Thr Val Val Ala Val
           115                 120                 125
   Ile Asp Ala Gly Phe Asp Lys Asn His Glu Ala Trp Arg Leu Thr Asp
       130                 135                 140
   Lys Thr Lys Ala Arg Tyr Gln Ser Lys Glu Asp Leu Glu Lys Ala Lys
   145                 150                 155                 160
   Lys Glu His Gly Ile Thr Tyr Gly Glu Trp Val Asn Asp Lys Val Ala
                   165                 170                 175
   Tyr Tyr His Asp Tyr Ser Lys Asp Gly Lys Thr Ala Val Asp Gln Glu
               180                 185                 190
   His Gly Thr His Val Ser Gly Ile Leu Ser Gly Asn Ala Pro Ser Glu
           195                 200                 205
   Thr Lys Glu Pro Tyr Arg Leu Glu Gly Ala Met Pro Glu Ala Gln Leu
       210                 215                 220
   Leu Leu Met Arg Val Glu Ile Val Asn Gly Leu Ala Asp Tyr Ala Arg
   225                 230                 235                 240
   Asn Tyr Ala Gln Ala Ile Arg Asp Ala Val Asn Leu Gly Ala Lys Val
                   245                 250                 255
   Ile Asn Met Ser Phe Gly Asn Ala Ala Leu Ala Tyr Ala Asn Leu Pro
               260                 265                 270
   Asp Glu Thr Lys Lys Ala Phe Asp Tyr Ala Lys Ser Lys Gly Val Ser
           275                 280                 285
   Ile Val Thr Ser Ala Gly Asn Asp Ser Ser Phe Gly Gly Lys Thr Arg
       290                 295                 300
   Leu Pro Leu Ala Asp His Pro Asp Tyr Gly Val Val Gly Thr Pro Ala
   305                 310                 315                 320
   Ala Ala Asp Ser Thr Leu Thr Val Ala Ser Tyr Ser Pro Asp Lys Gln
                   325                 330                 335
   Leu Thr Glu Thr Ala Met Val Lys Thr Asp Asp Gln Gln Asp Lys Glu
               340                 345                 350
   Met Pro Val Leu Ser Thr Asn Arg Phe Glu Pro Asn Lys Ala Tyr Asp
           355                 360                 365
   Tyr Ala Tyr Ala Asn Arg Gly Met Lys Glu Asp Asp Phe Lys Asp Val
       370                 375                 380
   Lys Gly Lys Ile Ala Leu Ile Glu Arg Gly Asp Ile Asp Phe Lys Asp
   385                 390                 395                 400
   Lys Val Ala Asn Ala Lys Lys Ala Gly Ala Val Gly Val Leu Ile Tyr
                   405                 410                 415
   Asp Asn Gln Asp Lys Gly Phe Pro Ile Glu Leu Pro Asn Val Asp Gln
               420                 425                 430
   Met Pro Ala Ala Phe Ile Ser Arg Lys Asp Gly Leu Leu Leu Lys Asp
           435                 440                 445
   Asn Pro Gln Lys Thr Ile Thr Phe Asn Ala Thr Pro Lys Val Leu Pro
       450                 455                 460
   Thr Ala Ser Gly Thr Lys Leu Ser Arg Phe Ser Ser Trp Gly Leu Thr
   465                 470                 475                 480
   Ala Asp Gly Asn Ile Lys Pro Asp Ile Ala Ala Pro Gly Gln Asp Ile
                   485                 490                 495
   Leu Ser Ser Val Ala Asn Asn Lys Tyr Ala Lys Leu Ser Gly Thr Ser
   500 505 510
   Met Ser Ala Pro Leu Val Ala Gly Ile Met Gly Leu Leu Gln Lys Gln
   515 520 525
   Tyr Glu Thr Gln Tyr Pro Asp Met Thr Pro Ser Glu Arg Leu Asp Leu
   530 535 540
   Ala Lys Lys Val Leu Met Ser Ser Ala Thr Ala Leu Tyr Asp Glu Asp
   545 550 555 560
   Glu Lys Ala Tyr Phe Ser Pro Arg Gln Gln Gly Ala Gly Ala Val Asp
   565 570 575
   Ala Lys Lys Ala Ser Ala Ala Thr Met Tyr Val Thr Asp Lys Asp Asn
   580 585 590
   Thr Ser Ser Lys Val His Leu Asn Asn Val Ser Asp Lys Phe Glu Val
   595 600 605
   Thr Val Thr Val His Asn Lys Ser Asp Lys Pro Gln Glu Leu Tyr Tyr
   610 615 620
   Gln Ala Thr Val Gln Thr Asp Lys Val Asp Gly Lys His Phe Ala Leu
   625 630 635 640
   Ala Pro Lys Val Leu Tyr Glu Ala Ser Trp Gln Lys Ile Thr Ile Pro
   645 650 655
   Ala Asn Ser Ser Lys Gln Val Thr Val Pro Ile Asp Ala Ser Arg Phe
   660 665 670
   Ser Lys Asp Leu Leu Ala Gln Met Lys Asn Gly Tyr Phe Leu Glu Gly
   675 680 685
   Phe Val Arg Phe Lys Gln Asp Pro Thr Lys Glu Glu Leu Met Ser Ile
   690 695 700
   Pro Tyr Ile Gly Phe Arg Gly Asp Phe Gly Asn Leu Ser Ala Val Glu
   705 710 715 720
   Lys Pro Ile Tyr Asp Ser Lys Asp Gly Ser Ser Tyr Tyr His Glu Ala
   725 730 735
   Asn Ser Asp Ala Lys Asp Gln Leu Asp Gly Asp Gly Leu Gln Phe Tyr
   740 745 750
   Ala Leu Lys Asn Asn Phe Thr Ala Leu Thr Thr Glu Ser Asn Pro Trp
   755 760 765
   Thr Ile Ile Lys Ala Val Lys Glu Gly Val Glu Asn Ile Glu Asp Ile
   770 775 780
   Glu Ser Ser Glu Ile Thr Glu Thr Ile Phe Ala Gly Thr Phe Ala Lys
   785 790 795 800
   Gln Asp Asp Asp Ser His Tyr Tyr Ile His Arg His Ala Asn Gly Glu
   805 810 815
   Pro Tyr Ala Ala Ile Ser Pro Asn Gly Asp Gly Asn Arg Asp Tyr Val
   820 825 830
   Gln Phe Gln Gly Thr Phe Leu Arg Asn Ala Lys Asn Leu Val Ala Glu
   835 840 845
   Val Leu Asp Lys Glu Gly Asn Val Val Trp Thr Ser Glu Val Thr Glu
   850 855 860
   Gln Val Val Lys Asn Tyr Asn Asn Asp Leu Ala Ser Thr Leu Gly Ser
   865 870 875 880
   Thr Arg Phe Glu Lys Thr Arg Trp Asp Gly Lys Asp Lys Asp Gly Lys
   885 890 895
   Val Val Ala Asn Gly Thr Tyr Thr Tyr Arg Val Arg Tyr Thr Pro Ile
   900 905 910
   Ser Ser Gly Ala Lys Glu Gln His Thr Asp Phe Asp Val Ile Val Asp
   915 920 925
   Asn Thr Thr Pro Glu Val Ala Thr Ser Ala Thr Phe Ser Thr Glu Asp
   930 935 940
   Arg Arg Leu Thr Leu Ala Ser Lys Pro Lys Thr Ser Gln Pro Val Tyr
   945 950 955 960
   Arg Glu Arg Ile Ala Tyr Thr Tyr Met Asp Glu Asp Leu Pro Thr Thr
   965 970 975
   Glu Tyr Ile Ser Pro Asn Glu Asp Gly Thr Phe Thr Leu Pro Glu Glu
   980 985 990
   Ala Glu Thr Met Glu Gly Ala Thr Val Pro Leu Lys Met Ser Asp Phe
   995 1000 1005
   Thr Tyr Val Val Glu Asp Met Ala Gly Asn Ile Thr Tyr Thr Pro Val
   1010 1015 1020
   Thr Lys Leu Leu Glu Gly His Ser Asn Lys Pro Glu Gln Asp Gly Ser
   1025 1030 1035 1040
   Gly Gln Thr Pro Asp Lys Lys Pro Glu Ala Lys Pro Glu Gln Asp Gly
   1045 1050 1055
   Ser Asp Gln Ala Pro Asp Lys Lys Pro Glu Ala Lys Pro Glu Gln Asp
   1060 1065 1070
   Gly Ser Gly Gln Thr Pro Asp Lys Lys Pro Glu Thr Lys Pro Glu Lys
           1075                1080                1085
   Asp Ser Ser Gly Gln Thr Pro Gly Lys Thr Pro Gln Lys Gly Gln Pro
       1090                1095                1100
   Ser Arg Thr Leu Glu Lys Arg Ser Ser Lys Arg Ala Leu Ala Thr Lys
   1105                1110                1115                1120
   Ala Ser Thr Arg Asp Gln Leu Pro Thr Thr Asn Asp Lys Asp Thr Asn
                   1125                1130                1135
   Arg Leu His Leu Leu Lys Leu Val Met Thr Thr Phe Phe Phe Gly Leu
               1140                1145                1150
   Val Ala His Ile Phe Lys Thr Lys Arg Gln Lys Glu Thr Lys Lys
           1155                1160                1165
(2) INFORMATION FOR SEQ ID NO: 3:
   (i) SEQUENCE CHARACTERISTICS:
      (A) LENGTH: 1150 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 3:
   Leu Arg Lys Lys Gln Lys Leu Pro Phe Asp Lys Leu Ala Ile Ala Leu
   1               5                   10                  15
   Met Ser Thr Ser Ile Leu Leu Asn Ala Gln Ser Asp Ile Lys Ala Asn
               20                  25                  30
   Thr Val Thr Glu Asp Thr Pro Ala Thr Glu Gln Thr Val Glu Thr Pro
           35                  40                  45
   Gln Pro Thr Ala Val Ser Glu Glu Ala Pro Ser Ser Lys Glu Thr Lys
       50                  55                  60
   Thr Pro Gln Thr Pro Ser Asp Ala Gly Glu Thr Val Ala Asp Asp Ala
   65                  70                  75                  80
   Asn Asp Leu Ala Pro Gln Ala Pro Ala Lys Thr Ala Asp Thr Pro Ala
                   85                  90                  95
   Thr Ser Lys Ala Thr Ile Arg Asp Leu Asn Asp Pro Ser Gln Val Lys
               100                 105                 110
   Thr Leu Gln Glu Lys Ala Gly Lys Gly Ala Gly Thr Val Val Ala Val
           115                 120                 125
   Ile Asp Ala Gly Phe Asp Lys Asn His Glu Ala Trp Arg Leu Thr Asp
   130 135 140
   Lys Thr Lys Ala Arg Tyr Gln Ser Lys Glu Asp Leu Glu Lys Ala Lys
   145 150 155 160
   Lys Glu His Gly Ile Thr Tyr Gly Glu Trp Val Asn Asp Lys Val Ala
   165 170 175
   Tyr Tyr His Asp Tyr Ser Lys Asp Gly Lys Thr Ala Val Asp Gln Glu
   180 185 190
   His Gly Thr His Val Ser Gly Ile Leu Ser Gly Asn Ala Pro Ser Glu
   195 200 205
   Thr Lys Glu Pro Tyr Arg Leu Glu Gly Ala Met Pro Glu Ala Gln Leu
   210 215 220
   Leu Leu Met Arg Val Glu Ile Val Asn Gly Leu Ala Asp Tyr Ala Arg
   225 230 235 240
   Asn Tyr Ala Gln Ala Ile Arg Asp Ala Ile Asn Leu Gly Ala Lys Val
   245 250 255
   Ile Asn Met Ser Phe Gly Asn Ala Ala Leu Ala Tyr Ala Asn Leu Pro
   260 265 270
   Asp Glu Thr Lys Lys Ala Phe Asp Tyr Ala Lys Ser Lys Gly Val Ser
   275 280 285
   Ile Val Thr Ser Ala Gly Asn Asp Ser Ser Phe Gly Gly Lys Thr Arg
   290 295 300
   Leu Pro Leu Ala Asp His Pro Asp Tyr Gly Val Val Gly Thr Pro Ala
   305 310 315 320
   Ala Ala Asp Ser Thr Leu Thr Val Ala Ser Tyr Ser Pro Asp Lys Gln
   325 330 335
   Leu Thr Glu Thr Val Arg Val Lys Thr Ala Asp Gln Gln Asp Lys Glu
   340 345 350
   Met Pro Val Leu Ser Thr Asn Arg Phe Glu Pro Asn Lys Ala Tyr Asp
   355 360 365
   Tyr Ala Tyr Ala Asn Arg Gly Thr Lys Glu Asp Asp Phe Lys Asp Val
   370 375 380
   Lys Gly Lys Ile Ala Leu Ile Glu Arg Gly Asp Ile Asp Phe Lys Asp
   385 390 395 400
   Lys Ile Ala Lys Ala Lys Lys Ala Gly Ala Val Gly Val Leu Ile Tyr
   405 410 415
   Asp Asn Gln Asp Lys Gly Phe Pro Ile Glu Leu Pro Asn Val Asp Gln
   420 425 430
   Met Pro Ala Ala Phe Ile Ser Arg Lys Asp Gly Leu Leu Leu Lys Asp
   435 440 445
   Asn Pro Gln Lys Thr Ile Thr Phe Asn Ala Thr Pro Lys Val Leu Pro
   450 455 460
   Thr Ala Ser Gly Thr Lys Leu Ser Arg Phe Ser Ser Trp Gly Leu Thr
   465 470 475 480
   Ala Asp Gly Asn Ile Lys Pro Asp Ile Ala Ala Pro Gly Gln Asp Ile
   485 490 495
   Leu Ser Ser Val Ala Asn Asn Lys Tyr Ala Lys Leu Ser Gly Thr Ser
   500 505 510
   Met Ser Ala Pro Leu Val Ala Gly Ile Met Gly Leu Leu Gln Lys Gln
   515 520 525
   Tyr Glu Thr Gln Tyr Pro Asp Met Thr Pro Ser Glu Arg Leu Asp Leu
   530 535 540
   Ala Lys Lys Val Leu Met Ser Ser Ala Thr Ala Leu Tyr Asp Glu Asp
   545 550 555 560
   Glu Lys Ala Tyr Phe Ser Pro Arg Gln Gln Gly Ala Gly Ala Val Asp
   565 570 575
   Ala Lys Lys Ala Ser Ala Ala Thr Met Tyr Val Thr Asp Lys Asp Asn
   580 585 590
   Thr Ser Ser Lys Val His Leu Asn Asn Val Ser Asp Lys Phe Glu Val
   595 600 605
   Thr Val Asn Val His Asn Lys Ser Asp Lys Pro Gln Glu Leu Tyr Tyr
   610 615 620
   Gln Ala Thr Val Gln Thr Asp Lys Val Asp Gly Lys His Phe Ala Leu
   625 630 635 640
   Ala Pro Lys Val Leu Tyr Glu Ala Ser Trp Gln Lys Ile Thr Ile Pro
   645 650 655
   Ala Asn Ser Ser Lys Gln Val Thr Val Pro Ile Asp Ala Ser Arg Phe
   660 665 670
   Ser Lys Asp Leu Leu Ala Gln Met Lys Asn Gly Tyr Phe Leu Glu Gly
   675 680 685
   Phe Val Arg Phe Lys Gln Asp Pro Lys Lys Glu Glu Leu Met Ser Ile
   690 695 700
   Pro Tyr Ile Gly Phe Arg Gly Asp Phe Gly Asn Leu Ser Ala Leu Glu
   705 710 715 720
   Lys Pro Ile Tyr Asp Ser Lys Asp Gly Ser Ser Tyr Tyr His Glu Ala
   725 730 735
   Asn Ser Asp Ala Lys Asp Gln Leu Asp Gly Asp Gly Leu Gln Phe Tyr
   740 745 750
   Ala Leu Lys Asn Asn Phe Thr Ala Leu Thr Thr Glu Ser Asn Pro Trp
   755 760 765
   Thr Ile Ile Lys Ala Val Lys Glu Gly Val Glu Asn Ile Glu Asp Ile
   770 775 780
   Glu Ser Ser Glu Ile Thr Glu Thr Ile Leu Ala Gly Thr Phe Ala Lys
   785 790 795 800
   Gln Asp Asp Asp Ser His Tyr Tyr Ile His Arg His Ala Asn Gly Lys
   805 810 815
   Pro Tyr Ala Ala Ile Ser Pro Asn Gly Asp Gly Asn Arg Asp Tyr Val
   820 825 830
   Gln Phe Gln Gly Thr Phe Leu Arg Asn Ala Lys Asn Leu Val Ala Glu
   835 840 845
   Val Leu Asp Lys Glu Gly Asn Val Val Trp Thr Ser Glu Val Thr Glu
   850 855 860
   Gln Val Val Lys Asn Tyr Asn Asn Asp Leu Ala Ser Thr Leu Gly Ser
   865 870 875 880
   Thr Arg Phe Glu Lys Thr Arg Trp Asp Gly Lys Asp Lys Asp Gly Lys
   885 890 895
   Val Val Ala Asn Gly Thr Tyr Thr Tyr Arg Val Arg Tyr Thr Pro Ile
   900 905 910
   Ser Ser Gly Ala Lys Glu Gln His Thr Asp Phe Asp Val Ile Val Asp
   915 920 925
   Asn Thr Thr Pro Glu Val Ala Thr Ser Ala Thr Phe Ser Thr Glu Asp
   930 935 940
   Arg Arg Leu Thr Leu Ala Ser Lys Pro Lys Thr Ser Gln Pro Val Tyr
   945                 950                 955                 960
   Arg Glu Arg Ile Ala Tyr Thr Tyr Met Asp Glu Asp Leu Pro Thr Thr
                   965                 970                 975
   Glu Tyr Ile Ser Pro Asn Glu Asp Gly Thr Phe Thr Leu Pro Glu Glu
               980                 985                 990
   Ala Glu Thr Thr Glu Gly Ala Thr Val Pro Leu Lys Met Ser Asp Phe
           995                 1000                1005
   Thr Tyr Val Val Glu Asp Met Ala Gly Asn Ile Thr Tyr Thr Pro Val
       1010                1015                1020
   Thr Lys Leu Leu Glu Gly His Ser Asn Lys Pro Glu Gln Asp Gly Ser
   1025                1030                1035                1040
   Asp Gln Ala Pro Asp Lys Lys Pro Glu Ala Lys Pro Glu Gln Asp Gly
                   1045                1050                1055
   Ser Gly Gln Thr Pro Asp Lys Lys Thr Glu Thr Lys Pro Glu Lys Asp
               1060                1065                1070
   Ser Ser Gly Gln Thr Pro Gly Lys Thr Pro Gln Lys Gly Gln Pro Ser
           1075                1080                1085
   Arg Thr Leu Glu Lys Arg Ser Ser Lys Arg Ala Leu Ala Thr Lys Ala
       1090                1095                1100
   Ser Thr Arg Asp Gln Leu Pro Thr Thr Asn Asp Lys Asp Thr Asn Arg
   1105                1110                1115                1120
   Leu His Leu Leu Lys Leu Val Met Thr Thr Phe Phe Leu Gly Leu Val
                   1125                1130                1135
   Ala His Ile Phe Lys Thr Lys Arg Gln Lys Glu Thr Lys Lys
               1140                1145                1150
(2) INFORMATION FOR SEQ ID NO: 4:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 4:
   GGGGGGGAAT TCGTAGCGGG TATCATGGGA C                                  31
(2) INFORMATION FOR SEQ ID NO: 5:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 5:
   GGGGGGGAAT TCGGGTGCTG CAATATCTGG C 31
(2) INFORMATION FOR SEQ ID NO: 6:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 6:
(2) INFORMATION FOR SEQ ID NO: 7:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE:cDNA
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE:cDNA
   (xi) SEQUENCE DESCRIPTION: SEQ ID NO: 8:
   CCCCCCGGAT CCACCAAAAC CCCACAAACT C 31
(2) INFORMATION FOR SEQ ID NO: 9:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 9: